(19) Europäisches Patentamt
European Patent Office
Office européen des brevets

(11) **EP 4 183 315 A1**

(12) **EUROPEAN PATENT APPLICATION**

(43) Date of publication:
**24.05.2023 Bulletin 2023/21**

(21) Application number: **23150127.1**

(22) Date of filing: **01.06.2017**

(51) International Patent Classification (IPC):
**A61B 1/00** *(2006.01)*     **A61B 1/005** *(2006.01)*
**A61B 1/018** *(2006.01)*     **A61B 18/00** *(2006.01)*
**A61B 18/14** *(2006.01)*     **A61B 34/00** *(2016.01)*
**A61B 90/00** *(2016.01)*

(52) Cooperative Patent Classification (CPC):
**A61B 1/018; A61B 1/00006; A61B 1/00042;**
**A61B 1/0008; A61B 1/0016; A61B 1/0051;**
**A61B 1/0057; A61B 34/71;** A61B 18/1492;
A61B 2018/00982; A61B 2034/715;
A61B 2090/062; A61B 2090/067; A61B 2090/0807;
A61B 2090/0811

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **01.06.2016  US 201662344393 P**

(62) Document number(s) of the earlier application(s) in
accordance with Art. 76 EPC:
**17807124.7 / 3 463 038**

(71) Applicant: **EndoMaster Pte. Ltd.**
**Singapore 119844 (SG)**

(72) Inventor: **Yamamoto, Tomonori**
**119844 Singapore (SG)**

(74) Representative: **Høiberg P/S**
**Adelgade 12**
**1304 Copenhagen K (DK)**

Remarks:
This application was filed on 03-01-2023 as a
divisional application to the application mentioned
under INID code 62.

(54) **ENDOSCOPY SYSTEM COMPONENTS**

(57)     Endoscopy system components including an endoscopy apparatus are disclosed. The endoscopy apparatus includes an elongate member for insertion into a shaft of a transport endoscope, a surgical tool and a visible feature. The surgical tool is coupled to a distal end of the elongate member.and has an effector at an opposite end. The visible feature is provided on the elongate member, the surgical tool or both. The location of the visible feature is fixed relative to a roll orientation of the effector, so that a position of the visible feature during use indicates the roll orientation of the effector.

Figure 3A          Figure 3B

EP 4 183 315 A1

**Description**

**FIELD OF INVENTION**

Herein disclosed is various components of an endoscopy system.

**BACKGROUND**

An endoscopy system allows a user to examine the interior of a hollow organ or cavity of the body.

There are many components in such an endoscopy system. There are many challenges to ensure that these components function within optimal parameters to ensure that the endoscopy system performs the endoscopy procedure properly.

For instance, the endoscopy system has an endoscope which carries surgical tools that are used to perform an endoscopy procedure. These tools are actuated by a drive mechanism, where the drive mechanism has to be operated such that there is no slack in tendons of the surgical tool. There also has to be synchronisation between an input device that controls the operation of the tools in that any movement of the input device should result in a commensurate actuation of the surgical tool.

The endoscope is handheld during insertion into a hollow organ or cavity of the body. The roll orientation of the endoscope and that of the surgical tool have to be aligned. Also after insertion, a user has to ensure that a tip of the surgical tool is in a correct orientation before commencing with the endoscopy procedure.

The following discloses an endoscopy system that seeks to address the above challenges.

**SUMMARY OF THE INVENTION**

According to a first aspect, there is provided an endoscopy apparatus comprising: an elongate member for insertion into a shaft of a transport endoscope, a surgical tool coupled to a distal end of the elongate member, the surgical tool having an effector at an opposite end; and a visible feature provided on the elongate member, the surgical tool or both, the location of the visible feature being fixed relative to a roll orientation of the effector, so that a position of the visible feature during use indicates the roll orientation of the effector.According to a second aspect, there is provided an endoscopy surgical instrument controller for an endoscopy surgical instrument, the endoscopy surgical instrument comprising a driving motor; a following motor; a joint arrangement; a pulling tendon that couples the driving motor to the joint arrangement; and a pushing tendon that couples the following motor to the joint arrangement, wherein the joint arrangement is actuated by the driving motor withdrawing the pulling tendon and the following motor releasing the pushing tendon, the endoscopy surgical instrument controller comprising: at least one processor; and at least one memory including computer program code, the at least one memory and the computer program code configured to, with the at least one processor, cause the endoscopy surgical instrument controller at least to: establish a displacement range occurring at the pulling tendon, within which the pulling tendon experiences maximum tension from being withdrawn by the driving motor; determine whether a command received to actuate the joint arrangement causes the driving motor to withdraw a length of the pulling tendon that falls within the displacement range; and instruct the following motor to restrict the releasing of the pushing tendon when the command is received, whereby a length of the pushing tendon released by the following motor is less than a length of the pulling tendon withdrawn by the driving motor over the displacement range, so that the tension experienced in the pulling tendon is caused by an extension of the pulling tendon.

According to a third aspect, there is provided an endoscopy system comprising: an endoscopy surgical instrument comprising: a driving motor; a following motor; a joint arrangement; a pulling tendon that couples the driving motor to the joint arrangement; and a pushing tendon that couples the following motor to the joint arrangement, wherein the joint arrangement is actuated by the driving motor withdrawing the pulling tendon and the following motor releasing the pushing tendon; and an endoscopy surgical instrument controller coupled to the endoscopy surgical instrument, the endoscopy surgical instrument controller configured to: establish a displacement range occurring at the pulling tendon, within which the pulling tendon experiences maximum tension from being withdrawn by the driving motor; determine whether a command received to actuate the joint arrangement causes the driving motor to withdraw a length of the pulling tendon that falls within the displacement range; and instruct the following motor to restrict the releasing of the pushing tendon when the command is received, whereby a length of the pushing tendon released by the following motor is less than a length of the pulling tendon withdrawn by the driving motor over the displacement range, so that the tension experienced in the pulling tendon is caused by an extension of the pulling tendon.

According to a fourth aspect, there is provided an endoscopy surgical instrument controller of an endoscopy system, the endoscopy system comprising an endoscopy surgical instrument, the endoscopy surgical instrument comprising a drive mechanism; and a terminal joint actuated by the drive mechanism, the terminal joint being disposed at the distal end of the endoscopy surgical instrument; the endoscopy system further comprising an input device in electrical communication with the drive mechanism, whereby movement of the input device causes the actuation of the terminal joint, the endoscopy surgical instrument controller comprising: at least one processor; and at least one memory including computer program code, the at least one memory and the computer program code configured to, with the at least one processor, cause the endoscopy surgical instrument controller at least to: detect for a signal resulting from movement of the input device, the signal providing a Cartesian position in a master workspace to which the input device has been moved, the master workspace providing a boundary within which the input device can be moved; process the received Cartesian position against a database that comprises Cartesian positions for the master workspace; Cartesian positions for a slave workspace providing a boundary within which the terminal joint can be actuated; and a mapping table that maps each Cartesian position in the master workspace to at least one Cartesian position in the slave workspace; determine a matching Cartesian position in the slave workspace for the received Cartesian position; and command the drive mechanism to actuate the terminal joint to the matching Cartesian position in the slave workspace.

According to a fifth aspect, there is provided an endoscopy surgical instrument controller of an endoscopy system, the endoscopy system comprising an endoscopy surgical instrument, the endoscopy surgical instrument comprising a drive mechanism; and a terminal joint actuated by the drive mechanism, the terminal joint being disposed at the distal end of the endoscopy surgical instrument; the endoscopy system further comprising an input device in electrical communication with the drive mechanism, whereby movement of the input device causes the actuation of the terminal joint, the endoscopy surgical instrument controller comprising: at least one processor; and at least one memory including computer program code, the at least one memory and the computer program code configured to, with the at least one processor, cause the endoscopy surgical instrument controller at least to: create a mobile tracer inside a slave workspace providing a boundary within which the terminal joint can be actuated, the mobile tracer being configured to track the input device by shifting inside the slave workspace in response to the input device being moved; detect for a signal resulting from movement of the input device; shift the mobile tracer to a Cartesian position within the slave workspace, wherein a distance of the shift depends on a Cartesian position of the input device inside a master workspace before and after the movement of the input device, the master workspace providing a boundary within which the input device can be moved; and command the drive mechanism to actuate the terminal joint to the Cartesian position of the mobile tracer in the slave workspace after the shift.

According to a sixth aspect, there is provided an adaptor for coupling a motor shaft to actuate a tendon of an endoscopy surgical instrument, the adaptor comprising a housing; a drum around which the tendon winds, the drum being rotatably coupled to the housing; and an energy storage mechanism arranged to apply torque on the drum.

According to a seventh aspect, there is provided a transport endoscope docking station comprising: a base having an endoscope attachment surface for mounting a transport endoscope, the base further having a drive mechanism attachment surface for mounting a drive mechanism to actuate a robotic member carried by the transport endoscope; and a platform to which the base is rotatably coupled.

## BRIEF DESCRIPTION OF THE DRAWINGS

Example embodiments of the invention will be better understood and readily apparent to one of ordinary skill in the art from the following written description, by way of example only, and in conjunction with the drawings. The drawings are not necessarily to scale, emphasis instead generally being placed upon illustrating the principles of the invention, in which:

Figure 1 is a schematic illustration providing a perspective view of an endoscopy system.
Figure 2 is a schematic illustration of a slave system of the endoscopy system of Figure 1.
Figures 3A and 3B each show a schematic of a portion of an endoscopy surgical instrument of the endoscopy system of Figure 1.
Figure 4A shows a flowchart depicting steps to operate a driving motor and a following motor of the endoscopy system of Figure 1.
Figure 4B shows a modification of the flowchart of Figure 4A.
Figure 5A shows a commanded position trajectory applied to each of a driving motor and a following motor of the endoscopy system of Figure 1 when they are operated to actuate a joint arrangement to a commanded position.
Figure 5B shows a commanded position trajectory applied to each of the driving motor and a following motor of the endoscopy system of Figure 1 when they are operated to actuate a joint arrangement to a commanded position.
Figure 6 shows a perspective view of input devices located at a master section of the endoscopy system of Figure 1.
Figures 7 and 8 each show a schematic diagram of components of the endoscopy system of Figure 1 with which an endoscopy surgical instrument controller communicates when translating movement at the input device into movement of a specific joint of a robotic member of the endoscopy system of Figure 1.
Figure 9 shows a graph of torque limit of a driving motor or motors in a drive mechanism of the endoscopy system of Figure 1 against the penetration depth.

Figure 10 shows a drum around which a tendon of an endoscopy surgical instrument of the endoscopy system of Figure 1 winds.

Figure 11A shows a schematic of an implementation of actuating an effector using a drum where an energy storage mechanism of the drum of Figure 10 is absent.

Figure 11 B shows a schematic of an implementation of actuating an effector using a drum where an energy storage mechanism of the drum of Figure 10 is present.

Figure 12 shows a side view of a transport endoscope docking station of the endoscopy system of Figure 1.

Figure 13 shows a perspective view of a transport endoscope docking station of the endoscopy system of Figure 1.

Figure 14 shows a perspective view of two endoscopy apparatuses having visible position indicator features.

## DETAILED DESCRIPTION

In the following description, various embodiments are described with reference to the drawings, where like reference characters generally refer to the same parts throughout the different views.

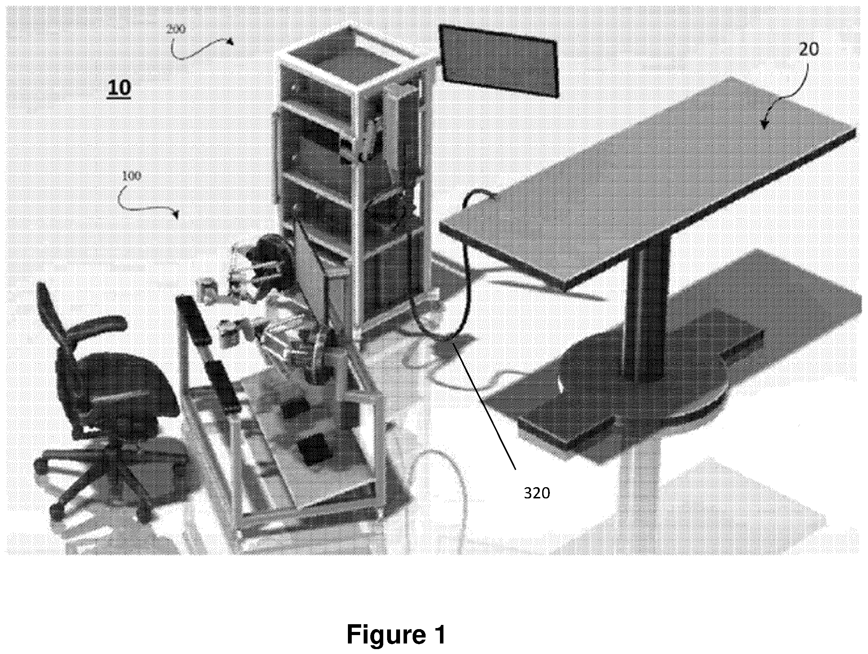

Figure 1 is a schematic illustration providing a perspective view of an endoscopy system 10. The endoscopy system 10 has a master or master-side section 100 having master-side elements and a slave or slave-side section 200 having slave-side elements.

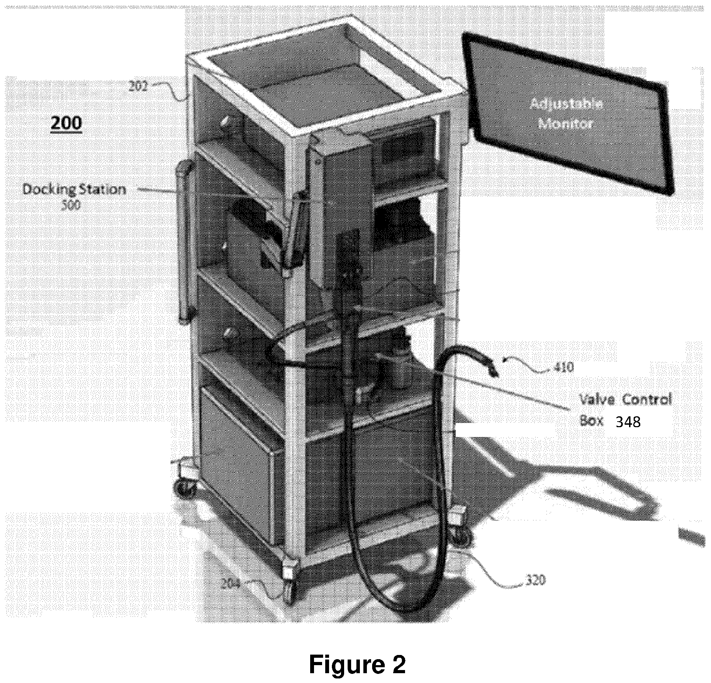

Figure 2 is a schematic illustration of the slave system 200 of the endoscopy system 10 of Figure 1. The slave system 200 has a patient-side cart, stand, or rack 202 configured for carrying at least some slave system elements. The patient-side cart 202 has a docking station 500 to which the transport endoscope 320 can be detached (e.g., mounted / docked and dismounted / undocked). The patient side cart 202 typically includes wheels 204 to facilitate easy portability and positioning of the slave system 200.

With reference to Figures 1 and 2, the master section 100 and the slave section 200 are configured for signal communication with each other such that the master section 100 can issue commands to the slave system 200 and the slave system 200 can precisely control, maneuver, manipulate, position, and/or operate, in response to master section 100 inputs, (a) a set of robotic members 410 carried or supported by a transport endoscope 320 of the slave section 200, the transport endoscope 320 having a flexible elongate shaft; (b) an imaging endoscope or imaging probe member carried or supported by the transport endoscope 320; and (c) a probe for surgical procedures, e.g. incision, dissection and/or hemostasis, by way of one or more of electrocauterisation (using an electrocautery), or lasing (using a laser), where electrical wiring connecting to the probe is carried or supported by the transport endoscope 320.

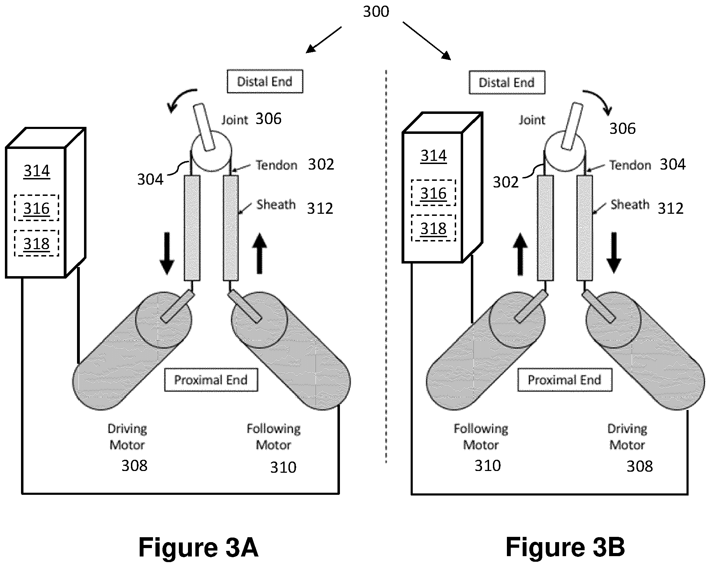

Robotic members 410 refer to apparatus that include effectors like arms or grippers that can grab and lift tissue. These effectors also include an electrocautery probe for dissection of tissue or for hemostasis. Actuation of the arms or grippers is brought about by a tendon of which two are shown in Figures 3A and 3B denoted using the reference numerals 302 and 304.

Figures 3A and 3B each show a schematic of a portion of an endoscopy surgical instrument 300. The endoscopy surgical instrument 300 includes a driving motor 308, a following motor 310, a joint arrangement 306 and a tendon pair 302 and 304. The driving motor 308 and the following motor 310 are disposed at a proximal end of the endoscopy surgical instrument, while the joint arrangement 306 is located at a distal end of the endoscopy surgical instrument 300. The driving motor 308 and the following motor 310 are detachable from the endoscopy surgical instrument 300.

The joint arrangement 306 may be a collection of joint segments which are mechanically joined to one another so that actuation of one of the joint segments leads to actuation of one or more of the other joint segments. The joint arrangement 306 provides the joints of the robotic members 410 of Figure 2, whereby each joint gives the robotic member 410 a degree of freedom of movement. Thus, actuation of the joint arrangement 306 results in the movement of the effectors of the robotic member 410, such as its arms, grippers or an electrocautery probe, allowing the robotic members 410 to grab and/or incise tissue.

Each tendon 302 and 304 of the tendon pair is enclosed by a flexible elongate sheath 312. The tendon pair includes a pulling tendon 304 and a pushing tendon 302. Each of the pulling tendon 304 and the pushing tendon 302 may be realised by a cable or any other line which is suitable to couple the driving motor 308 and the following motor 310 to the joint arrangement 306. The pulling tendon 304 couples the driving motor 308 to the joint arrangement 306, while the pushing tendon 302 couples the following motor 310 to the joint arrangement 306.

At a distal end, each of the tendons 302 and 304 is secured to the joint arrangement 306. For instance, if the pushing tendon 302 and the pulling tendon 304 are realised by a singular piece, the distal end of the pushing tendon 302 and the pulling tendon 304 are secured by wrapping around the joint arrangement 306. Alternatively, if the pushing tendon 302 and the pulling tendon 304 are realised by separate tendons, their distal ends are secured by being anchored to the joint arrangement 306.

At a proximal end, each of the tendons 302 and 304 is connected to the following motor 310 and the driving motor 308 such that the joint arrangement 306 is actuated by the driving motor 308 withdrawing the pulling tendon 304 and the following motor releasing the pushing tendon 302. This may be realised, for instance, by a shaft of the driving motor 308 rotating a drum connected thereto to wind the pulling tendon 304 around the drum. Similarly, a shaft of the following motor 310 rotates a drum connected thereto to unwind the pushing tendon 302 around the drum.

The driving motor 308, the following motor 310, the pulling tendon 304 and the pushing tendon 302 are so called because of their respective purposes during a phase of actuating the joint arrangement 306. To efficiently control the movement of the distal end joint arrangement 306 during each phase of the joint arrangement 306 actuation, one of the motors 308, 310 tensions its respective coupled tendon 304, 302, while the other motor relieves the tension by releasing its respectively coupled tendon. The motor that causes the tension is called the driving motor, while the motor that relieves the tension called the following motor. Since the tendon tension is effected by the driving motor pulling the tendon, the tendon that pulls the joint arrangement 306 is called the pulling tendon. On the other hand, since relief of tendon tension is effected by the following motor pushing the tendon, the tendon that pushes the joint arrangement 306 is called the pushing tendon.

It will thus be appreciated that the motors 308 and 310 will alternate between being a driving motor and a following motor, while the tendons 304 and 302 will alternate from being a pulling tendon and a pushing tendon during different phases of the joint arrangement 306 actuation. For example, Figure 3A depicts a phase where the joint arrangement 306 is rotated anti-clockwise, while Figure 3B depicts a phase where the joint arrangement 306 is rotated clockwise. The driving motor 308 in Figure 3A switches to the following motor 310 in Figure 3B, while the following motor 310 in Figure 3A switches to the driving motor 308 in Figure 3B. The pulling tendon 304 in Figure 3A switches to the pushing tendon 302 in Figure 3B, while the pushing tendon 302 in Figure 3A switches to the pulling tendon 304 in Figure 3B.

An endoscopy surgical instrument controller 314 is provided for controlling the operation of the driving motor 308 and the following motor 310. The endoscopy surgical instrument controller 314 may be integral or separate from the endoscopy system 10 of Figure 1. The endoscopy surgical instrument controller 314 may be realised by a generic computer or a specifically designed workstation integrated with the endoscopy system 10 of Figure 1 to which the endoscopy surgical instrument 300 is a component. The endoscopy surgical instrument controller 314 has at least one processor 316 and at least one memory 318, wherein the memory 318 stores computer program code to which the processor 316 executes when operating the driving motor 308 and the following motor 310. The endoscopy surgical instrument controller 314 includes an input port (not shown) to receive a command providing a position to which the joint arrangement 306 is to be actuated and a receiver module (not shown) to obtain the received command from the input port for the processor 316 to analyse. The endoscopy surgical instrument controller 314 further includes a transmitter module (not shown) to direct instructions to operate either the driving motor 308, the following motor 310 or both to cause the joint arrangement 306 to arrive at the commanded position and an output port (not shown) to relay these instructions to the driving motor 308, the following motor 310 or both. The receiver module and the transmitter module may be a hardware component or implemented by software.

The endoscopy surgical instrument controller 314 controls the operation of the driving motor 308 and the following motor 310 to maintain tension in the pulling tendon 304, the pushing tendon 302 or both. An optimal tension in the pulling tendon 304, the pushing tendon 302 or both has to be maintained when the joint arrangement 306 is actuated. The optimal tension in the pulling tendon 304 may be different from the optimal tension in the pushing tendon 302. This involves the following motor 310 hindering the release of the pushing tendon 302 so that the tension brought about from the driving motor 308 withdrawing the pulling tendon 304 is maintained. Hindering the release of the pushing tendon 302 also inhibits the distal joint arrangement 306 from being actuated to a commanded position through operation of the driving motor 308, while over release of the pushing tendon 302 introduces tendon slack. Thus, in controlling the operation of the driving motor 308 and the following motor 310, the endoscopy surgical instrument controller 314 has to identify an optimal set of operation parameters for instructing the driving motor 308, the following motor 310 or both when seeking to actuate the joint arrangement 306 to a desired position or a commanded position by the endoscopy surgical instrument controller 314.

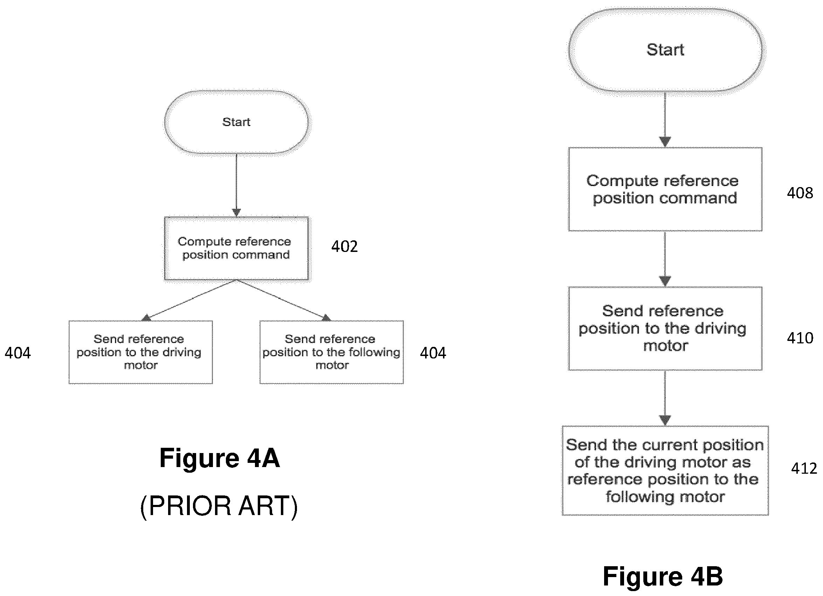

One existing approach is to synchronise the two motors 308 and 310, so that from the perspective of the endoscopy surgical instrument controller 314 they operate as if there were only one motor. Figure 4A shows a flowchart depicting steps for this existing approach.

In step 402, a control parameter that a motor (i.e. either the following motor 310 or the driving motor 308) needs to execute to actuate the joint arrangement 306 to a desired position is computed by the endoscopy surgical instrument controller 314. For example, this control parameter may be a motor shaft of the driving motor 308 having to undergo 10 revolutions to have the joint arrangement 306 rotate 10°. This parameter then becomes a reference position expressed, for example, in terms of encoder count of an encoder that monitors the operation of the motor, the encoder count being based on a position command in Joint Space or Cartesian Space that seeks to actuate the joint arrangement 306 to the desired position.

In step 404, the endoscopy surgical instrument controller 314 (see Figures 3A and 3B) sends an instruction containing the computed reference position to both the driving motor 308 and the following motor 310. However, when the driving motor 308 and the following motor 310 seek to execute the received reference position, complete synchronization does not occur because of different dynamics experienced at the pulling tendon 304 and the pushing tendon 302. Namely, a pulling action requires more motor torque than a releasing action and thus the driving motor 308 moves slower than the following motor 310. Further, should the distal end of the endoscopy surgical instrument 300 become constrained, due to an effector coupled to the joint arrangement 306 being blocked by an object or a joint in the joint arrangement 306 hitting a mechanical limit, the driving motor 308 moves even slower because it needs to stretch pulling tendon 304 to pull further. Another disadvantage is that even when the driving motor 308 reaches a torque limit and stops before the commanded reference position, the following motor 310 continues to the commanded reference motion and keeps releasing the pushing tendon 302. These factors create unnecessary tendon slack.

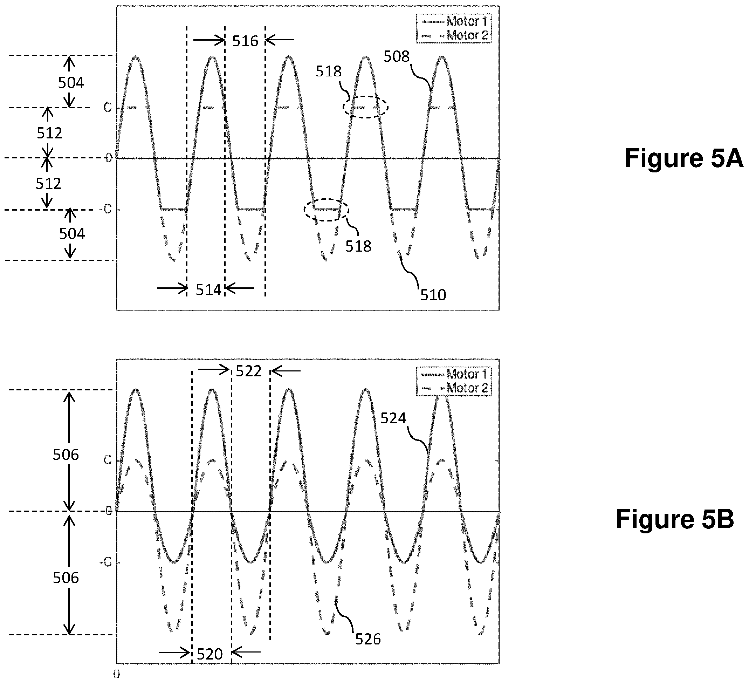

The endoscopy surgical instrument controller 314 addresses the shortfall of the approach illustrated in Figure 4A through adopting the approach brought about by the flowchart depicted in Figure 4B. The flowchart of Figure 4B is explained with reference to Figures 3A and 3B, along with Figures 5A and 5B.

The processor 316 of the endoscopy surgical instrument controller 314 executes computer program code stored in the memory 318 which causes the endoscopy surgical instrument controller 314 to establish a displacement range occurring at the pulling tendon 304, within which the pulling tendon 304 experiences maximum tension from being withdrawn by the driving motor 308. The displacement range falls within a distance the pulling tendon 304 travels or a length of the pulling tendon 304 withdrawn by the driving motor 308 when pulling the joint arrangement 306 to actuate to a commanded position. This displacement range may also be expressed in terms of encoder count.

In one implementation, the displacement range begins when the driving motor 308 commences operation to pull the joint arrangement 306 and ends when the joint arrangement 306 reaches its commanded position. This displacement range 506 is shown in Figure 5B to correspond to the range between 0 (zero) and a peak of a commanded position trajectory executed by the driving motor 308.

In another implementation, the displacement range begins around the point where a threshold of the following motor 310 is reached and ends when the joint arrangement 306 reaches its commanded position. This threshold of the following motor 310 is a calculated amount of the pushing tendon 302 that the following motor 310 releases before tendon slack occurs, which may cause the pushing tendon 302 to hop between grooves of a drum connected to a shaft of the following motor 310. This displacement range 504 is shown in Figure 5A to correspond to the range between C, the threshold where further release of the pushing tendon 302 by the following motor 310 causes tendon slack, and a peak of a commanded position trajectory executed by the driving motor 308.

The displacement range is established in step 408 of the flowchart of Figure 4B, along with a control parameter that a motor needs to execute to actuate the joint arrangement 306 to a desired position is computed by the endoscopy surgical instrument controller 314. Similar to step 402 of the flowchart of Figure 4A, this parameter then becomes a reference position expressed, for example, in terms of encoder count of an encoder that monitors the operation of the motor, the encoder count being based on a position command in Joint Space or Cartesian Space that seeks to actuate the joint arrangement 306 to the desired position.

In contrast to step 402 of the flowchart of Figure 4A, the reference position is only sent to the driving motor 308 in step 409 of the flowchart of Figure 4B. With the driving motor 308 being in receipt of the reference position, the driving motor 308 is informed of a length of the pulling tendon 304 to withdraw to actuate the joint arrangement 306 to a commanded position. Instead of sending the reference position to the following motor 310, a scaled extent of a current position of the driving motor 308 is sent to the following motor 310 instead in step 412. This effectively causes the following motor 310 to track the driving motor 308, for at least an interval of the driving motor 308 operation. Further detail of the step 412 is provided as follows.

The tracking of the driving motor 308 by the following motor 310 occurs when the endoscopy surgical instrument controller 314 receives a command that causes the driving motor 308 to withdraw a length of the pulling tendon 304 that falls within the displacement range (confer reference numeral 504 in Figure 5A and reference numeral 506 in Figure 5B). When such a command is received, the endoscopy surgical instrument controller 314 instructs the following motor 310 to restrict the releasing of the pushing tendon 302. A length of the pushing tendon 302 released by the following motor 310 is less than a length of the pulling tendon 304 withdrawn by the driving motor 308 over the displacement range, resulting in tension being experienced in the pulling tendon 304 caused by an extension of the pulling tendon 304.

The length of the pushing tendon 302 released by the following motor 310 being less than a length of the pulling tendon 304 withdrawn by the driving motor 308 occurs in the operation window where the joint arrangement 306 is being actuated to its commanded position. That is, over the entire duration of operating the driving motor 308 and the following motor 310 to actuate the joint arrangement 306 to its commanded position, the driving motor 308 experiences a higher encoder count than that of the following motor 310. This is implemented by having the driving motor 308 travel further than the following motor 310 through, for example, a motor shaft of the driving motor 308 revolving more than a motor shaft of the following motor 310, to prevent unnecessary tendon slack.

However, during this operation window, there are periods where the displacement of the pulling tendon 304 is approximately the same as the displacement of the pushing tendon 302, i.e. the withdrawn approximately the same as release. This occurs in the implementation shown in Figure 5A.

Figure 5A shows a commanded position trajectory applied to each of the driving motor 308 and the following motor 310 when they are operated to actuate the joint arrangement 306 to a commanded position, in accordance with a first implementation of having a length of the pushing tendon 302 released by the following motor 310 be less than a length of the pulling tendon 304 withdrawn by the driving motor 308. These trajectories are plotted in a graph of a commanded motor position against time.

It will be appreciated that which of the curves 508, 510 represents the driving motor 308 or the following motor 310 depends on a phase 514, 516. During the phase 514, the curve 508 represents a commanded position trajectory to the driving motor 308, while the curve 510 represents a commanded position trajectory to the following motor 310. During the next phase 516, the curve 510 represents a commanded position trajectory to the driving motor 308, while the curve 508 represents a commanded position trajectory to the following motor 310.

During at least a portion of the operation of the driving motor 308 and the following motor 310, the endoscopy surgical instrument controller 314 instructs the following motor 310 to have a length of the pushing tendon 302 being released be in accordance with a scaling factor when compared to a length of the pulling tendon 304 withdrawn by the driving motor 308. This portion is denoted reference numeral 512. During this portion 512, the driving motor 308 and the following motor 310 are synchronised to the extent that the driving motor 308 travels an approximately equal amount compared to the following motor 310, so that the scaling factor is at unity.

Instructions received by the endoscopy surgical instrument controller 314 during this portion 512 will be recognised by the endoscopy surgical instrument controller 314 as commands that cause the driving motor 310 to withdraw a length of the pulling tendon 304 that falls outside of the displacement range 504. Thus, before the endoscopy surgical instrument controller 314 receives a command that causes the driving motor 308 to withdraw a length of the pulling tendon 304 that falls within the displacement range 504, the driving motor 308 and the following motor 310 are operated so that the length of the pulling tendon 304 withdrawn is approximately the same as the length of the pushing tendon 302 released. However, after this command is received, the following motor 310 is then instructed to prevent release of the pushing tendon 302, which occurs over the displacement range 504 of Figure 5A, see circled portions 518 of the curves 508 and 510 indicating that the following motor 310 stops.

The instruction that the endoscopy surgical instrument controller 314 provides to the following motor 310 to restrict the releasing of the pushing tendon 302 over the displacement range 504 prevents tendon slack. Without this restriction, continuous release of the pushing tendon 302 causes tendon slack that can result in the proximal end of the pushing tendon 302 wrapping around a groove of a drum connected to a shaft of the following motor 310 to hop between grooves. This hopping between grooves is thus prevented from the implementation of Figure 5A, which limits the range of motion of the following motor 310, where this limitation is up to an extent of preventing the following motor 310 from releasing the pushing tendon 302.

In the implementation where the following motor 310 has a shaft that is coupled to a drum around which the pushing tendon 302 winds, if half a revolution of motion of the drum in the releasing direction starts creating tendon slack, a threshold C, -C (shown in Figure 5A) that corresponds to the half a revolution of the following motor 310 shaft is calculated. This threshold C, -C serves to limit the travel length of the following motor 310 to prevent the pulling tendon 304 hoping over situation described above. When the commanded position trajectory received by the driving motor 308 exceeds the threshold C, -C, the commanded position trajectory to the following motor 310 will be truncated to the threshold C, -C.

Figure 5B shows a commanded position trajectory applied to each of the driving motor 308 and the following motor 310 when they are operated to actuate the joint arrangement 306 to a commanded position, in accordance with a second implementation of having a length of the pushing tendon 302 released by the following motor 310 be less than a length of the pulling tendon 304 withdrawn by the driving motor 308. These trajectories are plotted in a graph of a commanded motor position against time.

Similar to Figure 5A, it will be appreciated that which of the curves 524, 526 of Figure 5B represents the driving motor 308 or the following motor 310 depends on a phase 520, 522. During the phase 520, the curve 524 represents a commanded position trajectory to the driving motor 308, while the curve 526 represents a commanded position trajectory to the following motor 310. During the next phase 522, the curve 526 represents a commanded position trajectory to the driving motor 308, while the curve 524 represents a commanded position trajectory to the following motor 310.

Similar to Figure 5A, during at least a portion of the operation of the driving motor 308 and the following motor 310, the endoscopy surgical instrument controller 314 instructs the following motor 310 to have a length of the pushing tendon 302 being released be in accordance with a scaling factor when compared to a length of the pulling tendon 304 withdrawn by the driving motor 308. However, in Figure 5B, this portion covers the entire duration of the operation of the driving motor 308 and the following motor 310. The scaling factor may alternatively be applied on the driving motor 308, so that the length of the pulling tendon 304 withdrawn is a multiple of the length of the pushing tendon 302 released that is greater than one.

Thus, in the implementation of Figure 5B, the displacement range 506 commences when the driving motor 308 starts the withdrawal of the pulling tendon 304. That is, when the endoscopy surgical instrument controller 314 is operated in accordance with Figure 5B, the endoscopy surgical instrument controller 314 receives the command that causes the driving motor 308 to withdraw a length of the pulling tendon 304 that falls within the displacement range 506 when actuation of the joint arrangement 306 commences. This is in contrast to the implementation of Figure 5A, where this command is received only after the driving motor 308 has withdrawn the pulling tendon 304 by a length corresponding to the portion 512 shown in Figure 5A.

The length of the pushing tendon 302 that is released by the following motor 310 is scaled compared to the length of the pulling tendon 304 that is withdrawn by the driving motor 308. However, in contrast to Figure 5A, the scaling is applied over the entire duration of the operation of the driving motor 308 and the following motor 310.

For long, flexible surgical instruments, it is challenging to estimate an accurate position of the end-effector coupled to the distal end of the joint arrangement 306 when there are no sensors at this distal end. Moreover, high payload on the distal end is more critical than high precision. This is because even if the end-effector moves as accurate as a user would like, without enough payload, the user would not be able to perform tasks to manipulate tissue through grabbing and lifting.

To solve these challenges and achieve maximum payload, the implementation of Figure 5B has the driving motor 308 pull the pulling tendon 304 as hard as possible while relieving tension through the following motor 310 release the pushing tendon 302, without creating slack. The implementation of Figure 5B does so by applying different scaling factors to the two motors, namely a bigger scaling factor for the driving motor 308 to achieve high payload and a smaller one for the following motor 310 to avoid tendon slack. The bigger scaling factor can be chosen by taking into consideration the motor torque limit. One method to choose the scaling factor is that when the biggest reference position for the driving motor 308 is commanded, the driving motor 308 just reaches the torque limit. The smaller scaling factor can be chosen such that when the biggest reference position for the following motor 310 is commanded, the following motor 310 just reaches the position threshold of C (or -C depending on a direction of motion).

In both the implementations of Figures 5A and 5B, the endoscopy surgical instrument controller 314 is further configured to detect for stoppage of the driving motor 308. This occurs at the peaks of the curves 508, 510, 524 and 526 occurring in the phases when each of them represent the driving motor 308. Each peak establishes a point within the displacement range 504, 506 where maximum tension is experienced by the pulling tendon 304.

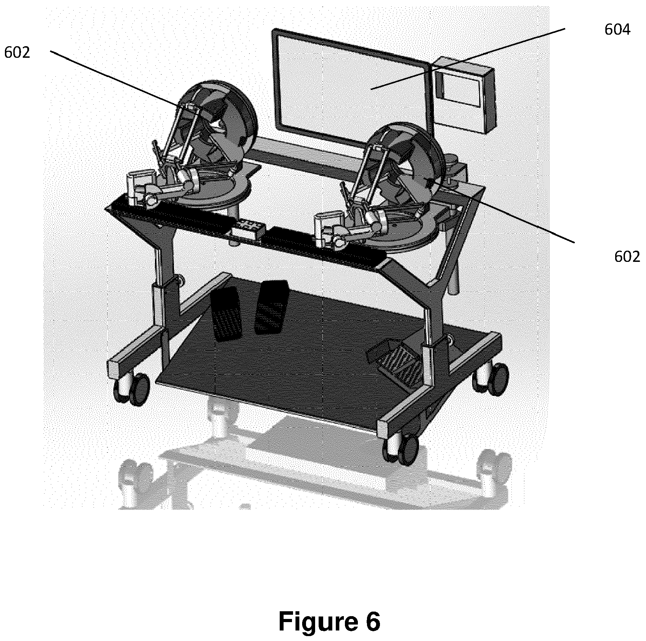

Figure 6 shows a perspective view of input devices 602 located at the master section 100 of the endoscopy system 10 (refer Figure 1). The input devices 602 allows movement control of the robotic members 410 (refer Figure 2) through controlling the actuation of one or more joints or a joint arrangement of the robotic members 410. With reference to Figures 3A and 3B, when these input devices 602 are moved, they provide command signals to the endoscopy surgical instrument controller 314 that operates the driving motor 308 and the following motor 310 to actuate the joint arrangement 306.

The input device 602 and a joint of the robotic member form a master-slave teleoperation system. When kinematically identical or equivalent devices are used for a master-slave teleoperation system, a simple controller to map from one joint of a master manipulator to a corresponding joint of a slave manipulator is easily implemented. However, when kinematically dissimilar devices are used, such as the input device 602 and a joint of the robotic member 410, the workspace of the master manipulator (namely the input device 602) and that of the slave manipulator (namely the joint of the robotic member 410) are different in size and shape.

For kinematically simple devices, i.e., manipulators with low degrees of freedom (DOFs), it is easy to map between the two workspaces. For a higher-DOF manipulator, mapping is not straightforward. The complexity increases when inverse kinematics is used where desired position and orientation of the master manipulator in three dimensional (3D) space need to be inversely calculated to reconstruct the same position and orientation of the slave manipulator with or without teleoperation scaling.

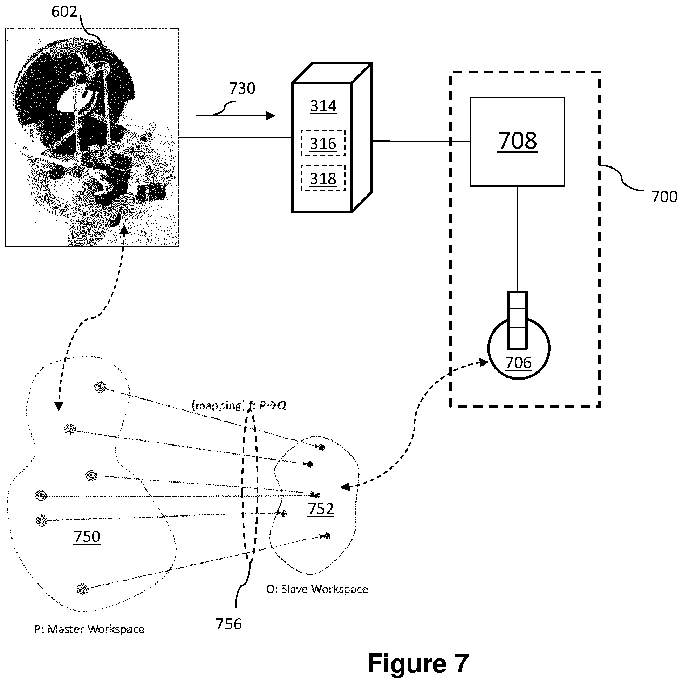

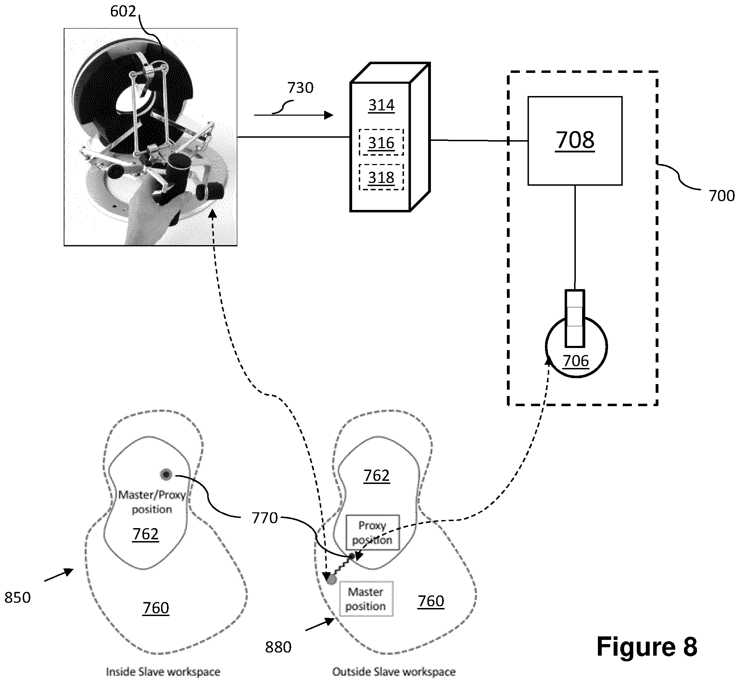

The endoscopy surgical instrument controller 314 solves such inverse kinematics problems, by ensuring the commanded posture is in the workspace of the slave manipulator as described below with reference to Figures 7 and 8 below.

Each of Figures 7 and 8 is a schematic diagram of components of the endoscopy system 10 of Figure 1 with which the endoscopy surgical instrument controller 314 communicates when translating movement at the input device 602 into movement of a specific joint of each of the robotic members 410. These components are an endoscopy surgical instrument 700 having a drive mechanism 708 and a terminal joint 706 actuated by the drive mechanism 708; and the input device 602.

The drive mechanism 708 is removably coupled to a proximal end of the endoscopy surgical instrument 700, while the terminal joint 706 is disposed at the distal end of the endoscopy surgical instrument 700. The drive mechanism 708 includes one or more motors or actuators that is coupled to the terminal joint 706 through a tendon and one or more motors or actuators to actuate each of the other joints in a joint arrangement to which the terminal joint 706 belongs. This drive mechanism 708 may therefore include the motors 308, 310 and the tendons 302, 304 of Figures 3A and 3B. In another implementation, the drive mechanism 708 may only use one motor to actuate each terminal joint 706. The terminal joint 706 is the joint of a robotic member 410 to which an effector is coupled, the effector being a surgical tool, such as any one of an arm, a gripper or an electrocautery probe.

The input device 602 is in electrical communication with the drive mechanism 708, whereby movement of the input device 602 causes the actuation of the terminal joint. As mentioned above, the input device 602 is located at the master section 100 of the endoscopy system 10.

In the implementation of Figure 7, the master workspace is projected onto the slave workspace. This is achieved as follows.

The memory 318 and the processor 316 of the endoscopy surgical instrument controller 314 are configured to cause the endoscopy surgical instrument controller 314 to detect for a signal 730 resulting from movement of the input device 602. The signal 730 provides a Cartesian position in a master workspace 750 to which the input device has been moved. This master workspace 750 provides a boundary within which the input device 602 can be moved, i.e. the master workspace stores all possible positions of the input device 602.

The received Cartesian position, which is extracted from the signal 730 by the endoscopy surgical instrument controller 314, is processed against a database (not shown) that comprises Cartesian positions for the master workspace 750; Cartesian positions for a slave workspace 752 providing a boundary within which the terminal joint 706 can be actuated; and a mapping table (not shown) that maps (see reference numeral 756) each Cartesian position in the master workspace 750 to at least one Cartesian position in the slave workspace 752.

The processing against the database is for the endoscopy surgical instrument controller 314 to transmit a command to the drive mechanism 708 to actuate the terminal joint 706 correspondingly to the detected movement of the input device 602. The extent of the corresponding movement of the terminal joint 706 is provided by the mapping table, since the mapping of each Cartesian position in the master workspace 750 to at least one Cartesian position in the slave workspace 752 serves to provide a position of the terminal joint 706 for each position of the input device 602. A unique mapping from the master workspace 750 to the slave workspace 752 is therefore established.

Thus after the received Cartesian position of the input device 602 is processed against the database, a matching Cartesian position in the slave workspace 752 for the received Cartesian position is determined. The endoscopy surgical instrument controller 314 then commands the drive mechanism 708 to actuate the terminal joint 706 to the matching Cartesian position in the slave workspace 752.

Since the master workspace 750 has a larger volume than that of the slave workspace 752, the mapping from the master workspace 750 to the slave workspace 752 is surjective; every point in the master workspace 750 is the value for at least one point in the slave workspace 752. That is, a plurality of the Cartesian positions in the master workspace 750 is mapped to a Cartesian position in the slave workspace 752.

The Cartesian position in the slave workspace 752 to which the plurality of the Cartesian positions in the master workspace 750 is mapped provides a closest matching Cartesian position in the slave workspace 752 for each of the plurality of the Cartesian positions in the master workspace 750. This allows for a closest point on the slave workspace 752 to be found for any point in the master workspace 750 at which the input device 602 is located, whereby the point in the master workspace 750 at which the input device 602 is located is then projected to this closest point on the slave workspace 752. Such a projection follows the equations

$$\forall q \in Q, \ \exists p \in P \quad \text{such that} \quad q = f(p) \quad \text{and} \quad \min \|q - p\|$$

where p and q are position in the master and slave workspaces 750, 752 and P and Q are master and slave workspaces 750, 752 respectively, and f is a function to map a point from a set P to a set Q under condition of the minimum distance between p and q.

Thus, the distance between a position p in the master workspace 750 and a position q in the slave workspace 752 is considered in mapping a point in the master workspace 750 to a point in the slave workspace 752. In one implementation, it is a Cartesian position in the slave workspace 752 that is closest to a Cartesian position in the master workspace 750 that the Cartesian position in the master workspace 750 is mapped. That is, a Cartesian position in the master workspace 750 is matched to a Cartesian position in the slave workspace 752 that is closest, when the slave workspace 750 is fitted into the master workspace 750.

In the implementation of Figure 8, operation of the input device 602 is tied to a proxy which always moves within the slave workspace. This is achieved as follows.

The memory 318 and the processor 316 of the endoscopy surgical instrument controller 314 are configured to cause the endoscopy surgical instrument controller 314 to create a mobile tracer 770 inside a slave workspace 762 providing a boundary within which the terminal joint 706 can be actuated. The mobile tracer 770 is configured to track the input device 602 by shifting inside the slave workspace 762 in response to the input device 602 being moved. Similar to Figure 7, the slave workspace 762 stores all possible positions of the terminal joint 706.

The endoscopy surgical instrument controller 314 then detects for a signal resulting from movement of the input device 602. When movement of the input device 602 is detected, the mobile tracer 770 is shifted to a Cartesian position within the slave workspace 762, wherein a distance of the shift depends on a Cartesian position of the input device 602 inside a master workspace 760 before and after the movement of the input device 602. Similar to Figure 7, the master workspace 760 provides a boundary within which the input device 602 can be moved, i.e. the master workspace 760 stores all possible positions of the input device 602.

The distance the mobile tracer 770 shifts depends on the Cartesian position of the input device 602 relative to the Cartesian position of the mobile tracer 770.

For instance the scenario 850 will occur if the master manipulator (i.e. the input device 602) moves inside the slave workspace 762, whereby the mobile tracer 770 position and orientation matches those of the master manipulator, shown by the Cartesian position of the input device 602 coinciding with the Cartesian position of the mobile tracer 770. That is, when the input device 602 moves inside the slave workspace 762, the mobile tracer 770 will follow the master position without being blocked by any obstacles and thus move approximately the same distance as the input device 602.

The scenario 880 occurs when the input device 602 goes out from the slave workspace 762. Since the mobile tracer 770 tracks the input device 602, the mobile tracer 770 is dragged by the input device 602, but remains inside the slave workspace 762. In this scenario, the mobile tracer 770 shifts less compared to the input device 602.

Thus the mobile tracer 770 is always confined within the slave workspace 762, where for any motion of the input device 602 in the master workspace 760, there will always be at least a degree of actuation of the terminal joint 706.

The endoscopy surgical instrument controller 314 commands the drive mechanism 708 to actuate the terminal joint 706 to the Cartesian position of the mobile tracer in the slave workspace after the shift.

In contrast to prior art techniques which focus on reducing the effects of time delay and tested for a low DOF system, the slave workspace 762 and the master workspace 760 are a higher DOF system.

In a high DOF system, orientation of an end-effector that is coupled to the distal end of the terminal joint 706 (or the joint arrangement 306 in the scenario of Figures 3A and 3B) is another inverse kinematics parameter to be solved. The endoscopy surgical instrument controller 314 solves this parameter by being configured to: synchronise an orientation of the input device 602 with an orientation of the joint arrangement 306 or the terminal joint 706, such that a change of orientation of the input device 602 results in a corresponding change in orientation of the joint arrangement 306 or the terminal joint 706. This approach is a direct mapping that fixes the orientation between the input device 602 and the joint arrangement 306 or the terminal joint.

The synchronisation may be done before the projection technique described with reference to Figure 7 is performed or before the proxy technique described with reference to Figure 8 is performed.

In the case of Figure 7, the orientation of the terminal joint 706 is first fixed to the orientation of the input device 602. The slave workspace 752 is then computed based on the commanded orientation of the input device 602. Unless a slave workspace 752 cannot be computed for a specific orientation of the input device 602, a slave workspace 752 is computed for every orientation position of the input device 602.

In the case of Figure 8, the slave workspace 752 is computed based on the commanded orientation of the input device 602. When the input device 602 moves inside the slave workspace 752, the position and orientation of the mobile tracer 770 matches those of the input device 602. Once the input device 602 goes out from the slave workspace 752, the mobile tracer 770 is dragged by the input device 602, but remains inside the slave workspace 752.

The endoscopy surgical instrument controller 314 is further configured to interrogate a database in which the slave workspace 762 and the master workspace 760 are stored when creating the mobile tracer 770. The endoscopy surgical instrument controller 314 also links the input device 602 to the mobile tracer 770 when configuring the mobile tracer 770 to track the input device 602, this linkage causing the mobile tracer 770 to be dragged by movement of the input device 602. The mobile tracer 770 moves adjacent to or along a perimeter of the slave workspace 762 when the input device 602 moves within a region of the master workspace 760 that is outside of the slave workspace 762.

For both Figures 7 and 8, the Cartesian position of the master workspace 750, 760 and that of the slave workspace 752, 762 provides a location in three-dimensional space. The master workspace 750, 760 has a larger volume than that of the slave workspace 752, 762.

Without any physical constraints, a user would not be aware of the boundary of the slave workspace 752, 762 and might operate the input device 602 in a region of the master workspace 750, 760 that is far away from the slave workspace 752, 762. This causes backlash-like effects when the user changes the direction of motion of the input device 602 and tries to go back from outside the slave workspace 752, 762, to inside the slave workspace 752, 762, but motion of the input device 602 does not result in any motion of the robotic members 410.

A virtual fixture can be used to create a physical constraint through the application of a resistance force, which can be proportional to the distance between the Cartesian position of the input device 602 in the master workspace 750, 760 and the Cartesian position of the terminal joint 706 in the slave workspace 752, 762. Such force can be based on a simple spring model or a damper-spring model. This virtual fixture may be realised by a feedback force module, which is a component of the endoscopy system 10 (see Figure 1). The feedback force module provides an indication that the terminal joint 706 is at a Cartesian position which is near or beyond the boundary of the slave workspace 752, 762. The feedback force module is coupled to the input device 602. The feedback force module serves to create a physical restraint by being configured to produce a resistive force that keeps the input device 602 within a region of the master workspace 750, 760 that corresponds to inside the boundary of the slave workspace 752, 762. The endoscopy surgical instrument controller 314 is further configured to transmit a signal to the feedback force module to increase the resistive force the further the input device 602 moves outside of the region of the master workspace 750, 760 that corresponds to the boundary of the slave workspace 752, 762.

The resistive force transmitted through the input device 602 is obvious to a user. When the resistive force increases, the user may have a very specific intention of the action being taken. For example, when the user operates the input device 602 while watching motion of the robotic member 401 through a monitor 604 (see Figure 6) that displays video images coming from the transport endoscope 320 (see Figure 2), the movement of the terminal joint 706 in the monitor 604 may be smaller than expected due to limited payload of the terminal joint 706. Hoping to increase the motion of the terminal joint 706, the user tends to move the input device 602 more, regardless of the increased resistive force.

The payload of the terminal joint 706 is directly tied with an amount of torque generated by the motors in the drive mechanism 708. Thus, the sensed resistive force or a penetration depth, being a measure of the distance between a Cartesian position of the terminal joint 706 and the boundary of the region of the master workspace 750, 760 that corresponds to the boundary of the slave workspace752, 762, can be used to adjust the motor torque limit.

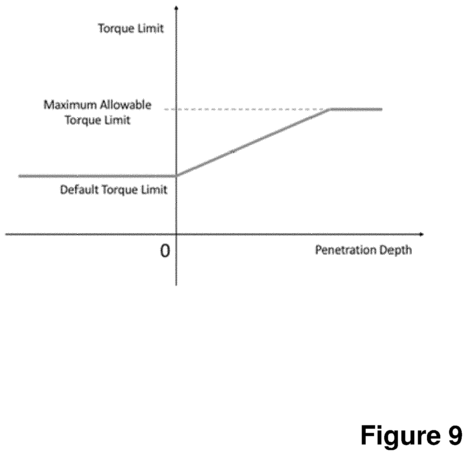

Figure 9 shows a graph of torque limit of the driving motor 308 or motors in the drive mechanism 708 against the penetration depth. The torque limit of the driving motor 308 or the motors in the drive mechanism 708 reflects the payload of the joint arrangement 306 or the terminal joint 706. A default torque is output under normal conditions, where the joint arrangement 306 or the terminal joint 706 is well reached anywhere within the slave workspace 752, 762 or displays sufficient payload to perform necessary tasks. With reference to Figures 3A, 3B and 7, the maximum allowable torque limit can be set taking into consideration the maximum torque the motors 308, 310 or the driving mechanism 708 can generate, breaking strength of the tendons 302, 304, among others.

Adjusting the torque limit on the fly offers a couple of advantages. First, the components of the surgical instrument 300, such as the tendons 302, 304, are less subject to wear and tear by using a torque limit that is smaller than that which causes maximum payload at the joint arrangement 306 or the terminal joint 706. This is because as long as the joint arrangement 306 or the terminal joint 706 moves as the user expects, there is no requirement for there to be a maximum payload at the distal end of the surgical instrument 300. Second, for more payload at the joint arrangement 306 or the terminal joint 706 on the distal end, the driving motor 308 or the driving mechanism 708 needs to pull as hard as possible. This elongates the pulling tendon 304 and results in more backlash-like effects when the driving motor 308 or the terminal joint 706 changes direction of motion. Therefore, under normal operation when the distal motion of the joint arrangement 306 or the driving mechanism 708 and payload is sufficient to perform a task, less backlash-like effects and wear and tear on the surgical instrument 300 components is preferred. However, if torque limit for the motors 308, 310 or the driving mechanism 708 is permanently fixed, the distal payload would be low. Therefore, reducing backlash-like effects and increasing distal payload are in mutual conflict. The endoscopy surgical instrument controller 314 being configured to adjust the torque limit applied by the driving motor 308 or the driving mechanism 708 to respectively actuate the joint arrangement 306 or the terminal joint 706, in response to the computed magnitude of the increase of the resistive force produced by the feedback force module, allows the striking of a balance in managing the degree of the backlash-like effect experienced as the distal payload is increased to the maximum allowable torque limit. When using a virtual spring for the feedback force module, the resistive force may be computed as follows. First, a penetration depth x of the distance between the Cartesian position of the input device 602 and the boundary of the slave workspace 752, 762 is computed. The resistive force F is then computed by $F = -k*x$, where k is the spring coefficient of the virtual spring.

While Figure 9 shows that a linear model is used, it will be appreciated that a polynomial model may be implemented. Once it starts pushing into the virtual fixtures, based on the distance, it keeps increasing the torque limit until it reaches the maximum allowable torque limit.

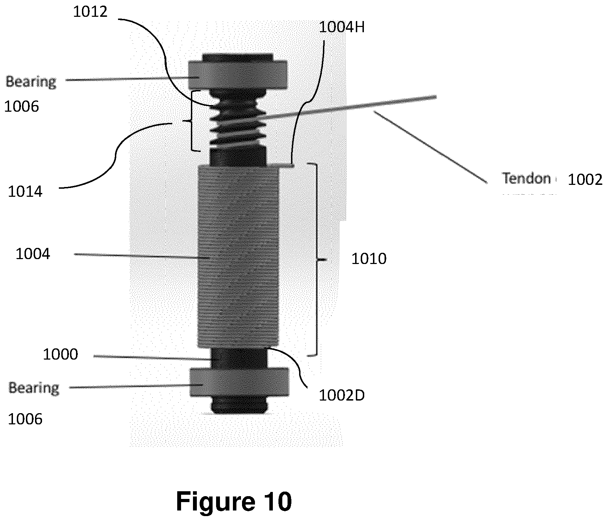

Figure 10 shows a drum 1000 around which a tendon 1002 of an endoscopy surgical instrument of the endoscopy system 10 of Figure 1 winds. For instance, either of the pulling tendon 304 or the pushing tendon 302 shown in Figures 3A and 3B can wind around the drum 1000, while the drum 1000 is actuated by one of the driving motor 308 and the following motor 310. Thus the drum 1000 may be a component of the endoscopy surgical instrument 300 described with reference to Figures 3A and 3B or it may be used with an endoscopy surgical instrument of another endoscopy system.

The drum 1000 is rotatably coupled to a housing, which is not shown for the sake of simplicity. The drum 1000 is rotatably coupled to the housing through a bearing 1006 located at each end of the drum 1000. This housing is part of an adaptor, which is also not shown for the sake of simplicity. The adaptor is detachable from a motor box, which for the endoscopy surgical instrument 300 of Figures 3A and 3B, contains the driving motor 308 and the following motor 310. Accordingly, this adaptor is for coupling a motor shaft to actuate a tendon of an endoscopy surgical instrument (or shortened form "instrument").

For a robotic endoscopy system, it is advantageous that the adaptor be operably detachable from the rest of the endoscopy system for cleaning and reprocessing, in the case where the endoscopy surgical instrument to which the adaptor belongs is reusable; or disposal, in the case of a single use endoscopy surgical instrument. For instrument designs that have service lives less than the rest of the endoscopy system, it is also advantageous to retain the driving actuators inside the rest of the endoscopy system, so as to keep the cost of the instrument portion low.

As described with reference to Figures 3A and 3B, a pair of counteracting tendons transmit force and motion from the actuator to the distal tip of the endoscopy surgical instrument. A level of tension has to be maintained in the tendons after the adaptor is detached from the actuators in the system. For instrument designs with more than one actuator per degree of freedom (DOF), tension in the tendons is lost when the instrument is detached from the actuators. Once tension in the tendons is lost, the tendons can become tangled inside the instrument, leading to tendon damage.

In WO2015142290, such tendon damage is addressed by implementing locking elements that automatically engage to fix the position of the tendons upon detachment of the instrument from the actuators, so that any tension present at the time of detachment is maintained. The said locking elements include friction or ratchet features. The said locking elements are then automatically mechanically withdrawn upon reattachment of the instrument onto the actuators.

Such locking elements have two primary disadvantages:
The first disadvantage is that by fixing the tendon positions at the time of detachment, the distal tip of the instrument becomes locked in its current position upon detachment. The instrument must pass through a lumen to reach the medical site. If the instrument distal tip was not straight at the time of detachment, or if an actuatable element of the distal tip, such as a grasping jaw, protrudes beyond the diameter of the lumen, it will be difficult or even impossible to remove the instrument from the lumen.

The second disadvantage is that prior to installation of the instrument onto the actuators, a user may unintentionally release the locking elements while handling the instrument, leading to loss of tendon tension and subsequent tendon damage.

With reference to Figure 10, to minimise or eliminate tendon damage that occurs in detachable instrument designs with more than one actuator per DOF, an adaptor for coupling a motor shaft to actuate the tendon 1002 of an endoscopy surgical instrument includes an energy storage mechanism 1004 arranged to apply torque on the drum 1000. It also seeks to eliminate the disadvantages of the current state of the art, by allowing the actuated elements of the instrument distal tip, such as end effectors, to remain flaccid for ease of insertion and extraction of the instrument through its lumen.

The energy storage mechanism 1004 may include any device that stores energy when the drum 1000 rotates from the release of tension in the tendon 1002 caused by detaching the adaptor from its actuators. The energy storage mechanism 1004 then tries to dissipate the energy by exerting a force in a direction opposite to the one causing the energy storage mechanism 1004 to store the energy.

Thus while the release of tension in the tendon 1002 causes the drum 1000 to rotate to unwind the tendon 1002, the energy storage mechanism 1004 applies a torque that prevents the unwinding of the tendon 1002. That is, the energy storage mechanism 1004 is positioned or arranged so as to apply the torque in a direction that winds the tendon 1002 around the drum 1000.

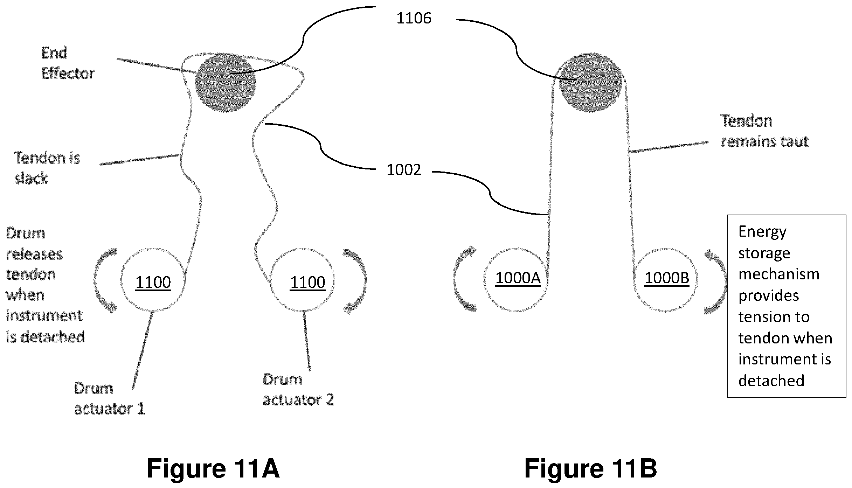

Figure 11A shows a schematic of an implementation where the energy storage mechanism 1004 is absent. When an adaptor housing drums 1100 is detached from its actuators, the release of tension causes the tendon 1002 to become slack since there is no device in these drums 1100 to prevent the release of tension.

On the other hand, Figure 11B shows a schematic of an implementation where the energy storage mechanism 1004 is present. When an adaptor housing drums 1000A, 1000B is detached from its actuators, the tendon 1002 remains sufficiently tensioned by the torque provided by the energy storage mechanism 1004 on each of the drums 1000A, 1000B. The tendon 1002 therefore remains taut. It will be appreciated that to effectively pre-tension the tendon 1002, the energy storage mechanism 1004 is arranged or positioned in each of the drums 1000A such that the torque applied on the drum 1000A is in a direction opposite to the torque applied on the drum 1000B. Thus, the addition of the energy storage mechanism 1004 alleviates tangling of the tendon 1002 within the endoscopy surgical instrument.

Returning to Figure 10, there are several ways in which the energy storage mechanism 1004 is secured to the drum 1000 and the housing of the adaptor. For example, one end 1004D of the energy storage mechanism 1004 is coupled to the drum 1000 and an opposite end 1004H of the energy storage mechanism 1004 is coupled to the housing.

Similarly, there are several possible locations of the energy storage mechanism 1004. For example, the energy storage mechanism 1004 is disposed around a portion of the drum 1000. Alternatively, the energy storage mechanism 1004 is disposed at either end of the drum 1000.

In one implementation (not shown), the energy storage mechanism 1004 is a hydraulic device, whereby the unwinding of the tendon 1002 from detachment of the adaptor pressurises hydraulics in the hydraulic device. The hydraulic device then seeks to relieve this pressure by applying a force in a direction opposite to the one pressurising the hydraulics.

In another implementation, the energy storage mechanism 1004 is a resiliently flexible member, whereby the unwinding of the tendon 1002 from detachment of the adaptor deforms the resiliently flexible member. The resiliently flexible member then seeks to return to its original shape by applying a force in a direction opposite to the one causing the deformation.

In the implementation shown in Figure 10, the energy storage mechanism 1004 is one realisation of a resiliently flexible member, namely a torsion spring. Further, the torsion spring used in Figure 10 is of a coil configuration. However, other configurations such as flat spiral, coil or axially twisted are possible.

The torsion spring on the drum 1000 is designed such that it provides minimal tendon tension to maintain orderly tendon 1002 wrapping around the drum 1000 when the endoscopy surgical instrument is unplugged from its actuators located in a motor box. This is to keep the tendon 1002 from hopping off the drum 1000 which could cause tangling when the instrument is unplugged from the motor box in a two motor per DOF system.

From experimental data, an optimal torque applied by the torsion spring on the tendon 1002 was determined to be around 0.5N to 3N. If the torque applied by the torsion spring leads to a pre-tension force that is too high, it would interfere with rotation of the drum 1000 by, for example, the driving motor 308 or the following motor 310 of Figures 3A and 3B.

Further, the torsion spring is designed to have the pretension as constant as possible over the endoscopy surgical instrument range of motion, which could be 0.25 to 2.0 revolutions of the drum 1000, depending on the tendon 1002 travel and the drum 1000 diameter. In order to achieve a low torsion constant, the torsion spring is manufactured to have several coils, leading to a high fineness ratio. It was found that a fineness ratio (see reference numeral 1010) of at least 14 produced a low torsion constant over the range of motion of the drum 1000.

To further facilitate orderly tendon 1000 wrapping around the drum 1000, the drum 1000 has at least one groove 1012 to which the tendon 1002 engages. The groove 1012 is provided on a portion of the drum 1000. Optionally, the groove 1012 extends along the diameter of the drum 1000. In the case where there is a plurality of grooves 1012, such as the case shown in Figure 10, a screw thread 1014 is formed on the drum 1000. The screw thread 1014 provides a plurality of the grooves 1012 to which the tendon 1002 engages.

In a typical endoscopic procedure, a transport endoscope (confer transport endoscope 320 shown in Figure 2) is handheld during the insertion phase and the transport endoscope (or shortened form "endoscope") is rolled along its elongate axis during the endoscopic procedure to orient the view of its distally disposed camera and its adjacent end effectors to a preferred orientation relative to a medical site. For example, in Endoscopic Submucosal Dissection procedures, it is common practice to rotate the endoscope 320 about its elongate axis until the tissue to be excised lies along the 6 o'clock position in the camera image, before starting the procedure. During such a hand held phase, manipulating the endoscope to the desired roll orientation is straightforward.

However, after reaching the desired medical site and after positioning the endoscope in a preferred roll orientation, time is needed to align the hand held endoscope and its supported robotic member (confer the robotic member 410 shown in Figure 2) when attaching the proximal end of the endoscope and the robotic member to the docking station. If the robotic member does not have a roll orientation degree of freedom to align itself to the roll orientation of the endoscope, the endoscope must be rolled to match the fixed roll orientation of the robotic member. This rotation of the endoscope causes the preferred distal roll orientation of the endoscope to be lost. In other words, the endoscope and the robotic member has to be further aligned in a roll orientation after attaching the endoscope and the robotic member to the docking station.

In order to solve the problems above, current robotic endoscopic systems utilize a clamp or an assistant holds the flexible elongate shaft of the endoscope member in the preferred orientation midway along the elongate member, nearby to where it enters the body. Subsequently, the user can twist the proximal end of the flexible elongate shaft in order to align the proximal roll orientation of the endoscope to the fixed roll orientation of the robotic member.

However, such a technique has two main disadvantages. Firstly, the flexible elongate shaft of the endoscope is designed to be torsionally stiff about its axis, so that roll motions and roll torques can be accurately transmitted from its proximal end to its distal end. Thus, introducing roll twist into the elongate shaft adds considerable stresses to the elongate member which may damage the components housed inside, such as effectors, tendons etc. Secondly, such a technique also introduces clinical risk. This technique leaves considerable stored energy in the twisted elongate shaft. If the elongate shaft roll orientation is not secured properly by the clamp or by the assistant, it may slip violently in the roll orientation, leading to a sudden and uncontrolled roll whip of the endoscope distal end. This motion could be dangerous to the patient, depending on the type of procedure and activities at the time of the whipping.

With reference to Figure 2, the docking station 500 prevents the loss of preferred distal tip orientation during docking and without the disadvantages of the aforementioned twisting technique in current systems. The endoscope docking station 500 allows the roll orientation of the robotic member 410 to match the roll orientation of the endoscope 320 during docking.

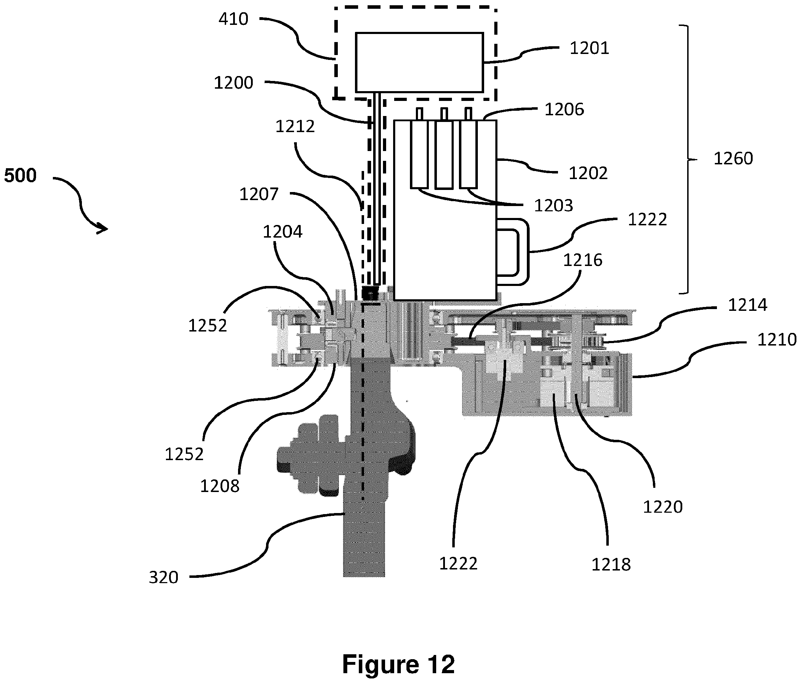

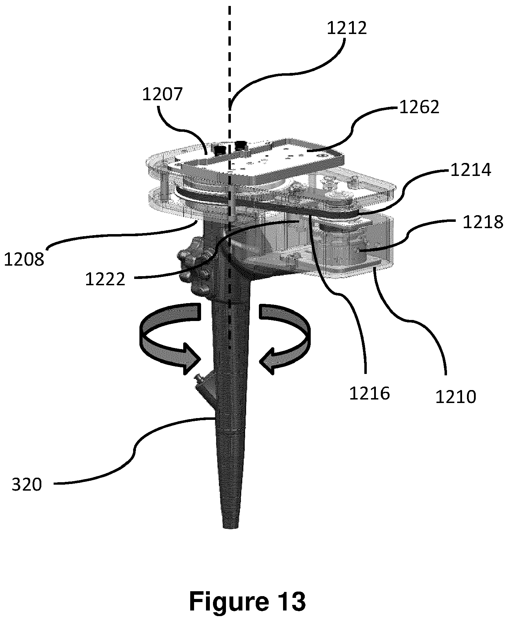

Figure 12 shows a side view of the transport endoscope docking station 500 while Figure 13 shows a perspective view of the transport endoscope docking station 500 of Figure 12 according to an example embodiment.

The transport endoscope docking station 500 includes a platform 1210 having a rotatable base 1204, i.e. the base 1204 is rotatably coupled to the platform 1210.

The base 1204 has an endoscope attachment surface 1208 for mounting the transport endoscope 320. The transport endoscope 320 is for carrying at least one robotic member 410, comprising a shaft 1200 and an adaptor 1201. The base 1204 also has a drive mechanism attachment surface 1207 for mounting a drive mechanism 1260 to actuate the robotic member 410 carried by the transport endoscope 320. The base 1204 is rotatably coupled about an axis 1212 perpendicular to a plane of the endoscope attachment surface 1208 of the base 1204 should the endoscope attachment surface 1208 be a planar surface.

The base 1204 includes a stand 1262 to which an actuator assembly of the drive mechanism 1260 is coupled. In Figure 12, the actuator assembly is realised by an actuator housing 1202 comprising a plurality of actuators 1203 configured to actuate the at least one robotic member 410. In Figures 12 and 13, the actuator housing 1202 and an adaptor 1201 that couples to an adaptor attachment surface 1206 of the actuator housing 1202 form part of the drive mechanism 1260. The adaptor 1201 is for coupling at least one of the actuators 1203 to the robotic member 410. A portion (such as the actuator housing 1202) of the drive mechanism 1260 may be integral with the base 1204 and a remainder (such as the adaptor 1201) of the drive mechanism 1260 is removably attachable to the integrated portion of the drive mechanism 1260.

After the transport endoscope 320 is attached to the endoscope attachment surface 1208, robotic members 410 may be introduced into the endoscope 320 to reach the work site. Following insertion of the robotic member shaft 1200 into the endoscope 320, the robotic member adaptor 1201 is attached to the adaptor attachment surface 1206. After attachment, the robotic members 410 and the endoscope 320 will rotate together with the base 1204 as an integrated unit. In other words, subsequent rotation of the base 1204 during the procedure will not result in any relative motion between the robotic members 410 and the endoscope 320. The robotic members 410 and the endoscope 320 can be rotated together as one unit to the desired rotational alignment relative to the medical site.

The transport endoscope docking station 500 may further comprise a rotary mechanism 1252 arranged to facilitate rotation of the base 1204 relative to the platform 1210. The rotary mechanism 1252 facilitates rotation by allowing the base 1204 to rotate smoothly relative to the platform 1210. The rotary mechanism 1252 may be realised using friction reducing elements, such as any one or more of a ball bearing arrangement, a roller bearing arrangement and a lubricated washer arrangement. In the embodiment shown in Figure 12, the rotary mechanism 1252 is realised by a ball bearing mechanism to facilitate the rotation of the base 1204.

The rotary mechanism 1252 is disposed between the base 1204 and the platform 1210. In the embodiment shown in Figure 12, the rotary mechanism 1252 is realised by two sets of ball bearing arrangements. One of the ball bearing arrangements is disposed between the base 1204 and a portion of the platform 1210 adjacent to where the base 1204 couples to the drive mechanism attachment surface 1206 of the base 1204. The other of the ball bearing arrangements Is disposed between the base 1204 and a portion of the platform 1210 adjacent to where the base 1204 couples to the endoscope attachment surface 1208 of the base 1204. However, it will be appreciated that the rotary mechanism 1252 may be realised by only a single ball bearing, roller bearing or lubricated washer arrangement, along a portion to where the base 1204 couples to the platform 1210. Accordingly, the rotary mechanism 1252 may be disposed between the base 1204 and the platform 1210 that is adjacent to the drive mechanism attachment surface of the base 1204, between the base 1204 and the platform 1210 that is adjacent to the endoscope attachment surface of the base 1204, or both.

The transport endoscope docking station 500 may include a locking mechanism 1218 arranged to lock the rotation of the base 1204 relative to the platform 1210. The locking mechanism 1218 may include an electrically activated device, such as a brake pad, a clamp and a latch and vault arrangement, configured to lock the base 1204 through frictional engagement, whereby rotation of the base 1204 is prevented.

The locking mechanism 1218 may be configured to lock the base 1204 when the locking mechanism 1218 is electrically inactive. In one implementation, the locking mechanism 1218 is designed such that, by default, it prevents rotation of the base 1204. This default state occurs when the locking mechanism 1218 is not operated to release the base 1204 for rotation. The release of the base 1204 is achieved through suitably operating an interface that controls the locking mechanism 1218, whereby the locking mechanism 1218 then becomes electrically active. The interface may be further configured to lock the base 1204 should the locking mechanism 1218 remain dormant for a period of time. This ensures safety from a clinical risk perspective by preventing the roll orientation of the base 1204 to be unintentionally shifted during an endoscopy procedure, since rotation of the base 1204 occurs only over a fraction of endoscopy procedure time.

A user may activate the locking mechanism 1218 to lock the base 1204 after roll orientation alignment is completed. After aligning the roll orientation of the endoscope 320 with the robotic member 410 (see Figure 2), the locking mechanism 1218 may be configured to automatically lock the base 1204, so that no further rotation of the base 1204 occurs should power be removed from the endoscopy system 10 (see Figure 1).

The transport endoscope docking station 500 includes a connector 1216 that couples the locking mechanism 1218 to the base 1204. The connector may be any one of a timing belt arrangement, a gear arrangement or an arm linkage. In the embodiment shown in Figure 12, the connector 1216 is realised by a timing belt arrangement comprising a belt and pulley arrangement. The pulley arrangement comprises at least two timing pulleys about which the belt is mounted. In addition, the connector 1216 includes a gear 1214 having a shaft 1220 which is coupled to the locking mechanism 1218, the belt coupling the locking mechanism 1218 to the base 1204.

During an endoscopic procedure, a robotic member is rotated so that it is at a desired position. As the endoscope transport 320 is connected to the endoscope attachment surface 1208 of the base 1204 together with the adaptor 1201 that is connected to the drive mechanism attachment surface 1206 of the base 1204, the rotary mechanism serves to adjust the roll of the transport endoscope 320 such that it is aligned with the roll orientation of the robotic member.

After docking of the transport endoscope 320 and the adapter, there may be unintentional rotary motion of the transport endoscope docking station 500 before the default state of the locking mechanism 1218 engages to lock the base 1204. Unintentional rotary motion of the transport endoscope docking station 500 may also occur when the user needs to unlock the transport endoscope 320 and adapter 1201 after the surgical procedure to an unlocked state. In order to limit such unintentional rotation, the rotary mechanism may include a damping mechanism 1222 to dampen rotation of the base 1204 to an acceptable speed. The damping mechanism 1222 may comprise a fluidic rotary damper, which in one implementation, is coupled to the connector 1216 coupling the locking mechanism 1218 to the base 1204 as shown in Figure 12. The fluidic rotary damper may comprise a fluid whereby the rotation of the connector 1216 is controlled and/or dampened by the fluid viscosity. In this way, rotation of the transport endoscope docking station 500 can be suitably controlled if the base 2014 rotation is unintentionally unlocked by the user.

In other embodiments, the damping mechanism 1222 may also comprise other types of rotary dampers that may control the rotation of the transport endoscope docking station 500. Examples of other rotary dampers may include either one of a rotary friction disk arrangement, a rotary friction gear rack arrangement, a pneumatic rotary damper and/or a visco-elastic rotary damper. Further, it may be appreciated that a rotational inertia of the base 1204 may be sufficient to dampen the rotation the transport endoscope docking station 500 such that a damping mechanism may not be necessary.

In addition, the transport endoscope docking station 500 may include a handle 1222 that may be attached to the actuator housing 1202 during docking of the endoscope as shown in Figure 12. Having the handle 1222 on the transport endoscope docking station 500 and in close proximity to the user may allow better manual control of the transport endoscope docking station 500 in its unlocked state.

An axis running through a centre of a proximal end of the adaptor 1201 may be aligned with the rotation axis 1212 of the base 1204. This vertical alignment prevents a rolling moment about the axis 1212 by an off-axis center-of-mass of the transport endoscope docking station 500. That is, if the centre-of-mass of the transport endoscope docking station 500 does not lie on the axis 1212, a rotational moment of the centre-of-mass about the axis 1212 is produced if the axis 1212 is not vertical. Such rotational moment will cause unintentional rotation of the base 1204. In addition, a vertically aligned axis 1212 will not cause the base 1204 to unintentionally rotate about the vertical axis 1212.

Alternatively, the center-of-mass of the transport endoscope docking station 500 may also be substantially close to the axis 1212, such that any remaining roll moment does not result in unacceptable whipping motions of the transport endoscope docking station 500 in the unlocked state.

Robotic medical tools, used to achieve medical purposes inside the body, are carried inside the transport endoscope 320. The robotic medical tools have an elongate member, sometimes called a shaft (compare the shaft 1200 of Figure 12, with a proximal end and a distal end. A robotic member (see robotic member 410 of Figure 2) is provided at the distal end of the elongate member and is the portion of the tool that is inserted inside the body through an incision, through a natural orifice, or through an auxiliary guide lumen to reach the medical site. The shaft 1200 may either be rigid or flexible.

Due to insertion through an incision, through a natural orifice, or through an auxiliary guide lumen, robotic medical tools typically have at least a roll degree of freedom and a translation degree of freedom. The roll degree of freedom is defined as the rotation of the tool about a longitudinal axis of the elongate member. The translation degree of freedom is defined as the movement of the tool along the longitudinal axis of the elongate member.

In order to minimize the incision size or in order to utilize small natural orifices, robotic actuators (compare the actuators 1203 of Figure 12) for controlling the robotic medical tool are often located externally to the patient. Where external actuators are used, the roll position and the translation position of the distal end of the elongate member are controlled by adjusting the roll position and the translation position of the proximal end. The elongate member cannot be perfectly torsionally stiff in the roll degree of freedom, giving rise to errors in the roll position of the distal end due to twisting of the elongate member about its longitudinal axis. Similarly, the elongate member cannot be perfectly stiff in the translation degree of freedom, giving rise to errors in the translation position of the distal end due to compression or stretch of the elongate member along its longitudinal axis.

These distal end position errors are particularly significant under the following conditions: when the elongate member is long or flexible; when the elongate member experiences friction when moving relative to the incision, relative to the natural orifice, or relative to an auxiliary lumen through which it passes to reach the medical site; and when large forces are exerted on the tool by the tissue with which it interacts to accomplish the medical purpose.

Distal end position errors are especially undesirable to the user when one degree of freedom is to be held in a fixed predetermined position for some length of time. One such example of this situation involves controlling the distal end roll orientation position such that robotic tool movement directions correspond to the commanded movement directions by the tool operator. For example, if the tool operator commands a bending articulation of the robotic tool distal tip in the upwards direction, the bending articulation direction of the distal end might be upwards and slightly left or upwards and slightly right, if there is an error in the distal end roll orientation. These errors are distracting and frustrating to the user.

Another situation in which distal position errors are important relates to instruments that are introduced to the medical site through an auxiliary guide lumen. In such arrangements, it is desirable that the tool operator be allowed to set a reference translation position prior to use. If the tool does not fully emerge from the guide lumen at the reference translation position, the guide lumen may interfere with operation of the tool, or the operation of the tool may damage the guide lumen. In the case of gastrointestinal endoscopes with integral auxiliary guide lumens, the distal end of the guide lumen is situated outside the integral camera's field of view. In these cases, it is difficult for the tool operator to compensate manually for distal tip translation errors in setting the reference translation position.

There are currently two main approaches to minimizing distal tip position errors. The first known strategy to minimize distal end position errors consists of making the elongate member as stiff as possible to minimize twist of the elongate member in the roll direction and minimize compression and stretch of the elongate member in the translation direction. However, such a strategy has several disadvantages. It may not be possible to make the elongate member stiffer when the elongate member must remain flexible in order to follow a non-straight path to the medical site. This is often the case with robotic tools that reach around sensitive anatomy, or those that follow the natural lumens of the body like the venous system, the urinary tract, the airway tract, or the gastrointestinal tract. Further, the stiffer elongate member often requires a larger outside diameter, which could make access to the medical site more invasive or difficult. In addition, the stiffer elongate member may require the use of exotic materials or exotic manufacturing methods that are economically infeasible.

The second known strategy consists of using a control system containing at least one sensor to measure the distal end positions and automatically compensate for distal end position errors with motion of the proximal end of the elongate member (as disclosed in WO2017048194). Such an approach has several disadvantages. Firstly, the sensor must detect the positions of the instrument distal end with a high degree of accuracy to avoid over compensation or under compensation of the position, leading to uncontrolled motion of the tool. Uncontrolled motion of the instrument could be significantly dangerous for the patient, depending on the function of the tool and the situation. Secondly, the sensor must reliably detect the position of the instrument distal end in order to be useful. A sensor that incorrectly senses the position even a small portion of the time will be frustrating to the user. There are many issues that make reliable position sensing difficult. For optical sensing methods, the tracked position target can be obscured by material in the medical environment, including bodily fluids or solids. Magnetic and electromagnetic position sensing methods must reject errors due to electromagnetically noisy environments. High voltage electrocautery tools, in particular, make reliable sensing difficult by these methods. Finally, adding a sensor or sensor target to the tool increases the cost of the endoscopy system.

The above shortcomings are addressed by having an endoscopy apparatus with highly visible position indicator features disposed on the elongate member. These visible position indicator features are located sufficiently adjacent to the distal end of the endoscopy apparatus. These visible position indicator features visually guide the user to align the tool to a predetermined position in at least one of the roll and translation degrees of freedom.

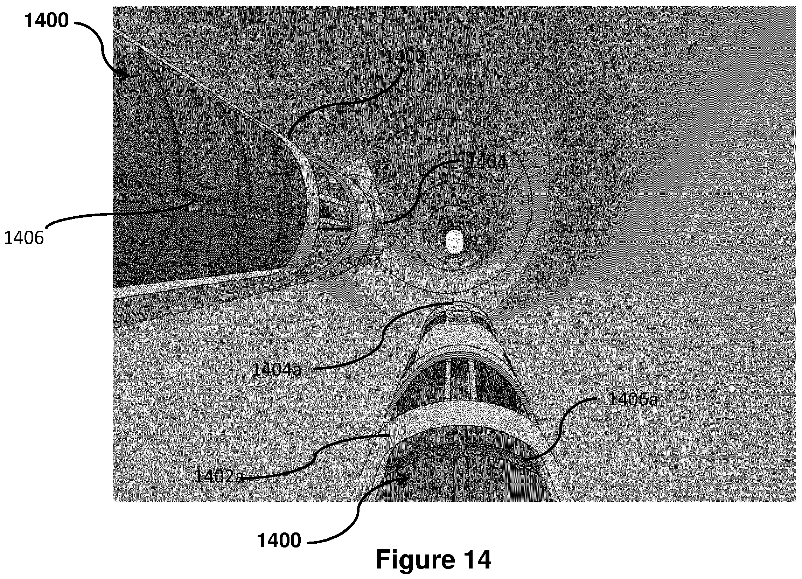

Figure 14 shows a perspective view of two endoscopy apparatuses 1400 having these visible position indicator features in accordance to an example embodiment. Each endoscopy apparatus 1400 comprises an elongate member (not shown) for insertion into a shaft of a transport endoscope (confer the transport endoscope 320 shown in Figures 12 and 13) and a surgical tool 1402, 1402a coupled to a distal end of the elongate member. The surgical tool 1402, 1402a has an effector 1404, 1404a at the opposite end of the surgical tool 1402, 1402a, i.e. the end opposite to where the surgical tool 1402, 1402a couples to the elongate member.

A visible feature 1406, 1406a is provided on the elongate member, the surgical tool 1402, 1402a or both. The location of the visible feature 1406, 1406a is fixed relative to a roll orientation of the effector 1404, 1404a, so that a position of the visible feature 1406, 1406a during use indicates the roll orientation of the effector 1404, 1404a.

The visible feature 1406, 1406a is any item that is visually distinguishable from a remainder of the structure on which the visible feature 1406, 1406a is located. For the visible feature 1406, 1406a to be visually distinguishable, in one implementation, it occupies only a portion of the area of the exterior surface of the elongate member or only a portion of the area of an exterior surface of the surgical tool 1402, 1402a. To this effect, the visible feature 1406, 1406a may extend over a portion along a length of the elongate member or the surgical tool 1402 or both. The visible feature 1406 may also extend over a portion along a cross-sectional perimeter of the elongate member or the surgical tool 1402 or both. The remainder of the exterior surface of the elongate member and the exterior surface of the surgical tool 1402, 1402a remains unaltered and is nondescript compared to the distinctiveness of the visible feature 1406, 1406a.

The visible feature 1406, 1406a provided on the elongate member or an exterior surface of the surgical tool 1402, 1402a may be formed on the material of the exterior surface of the elongate member or the exterior surface of the surgical tool 1402, 1402a through, for example, laser marking, embossing or surface texturing. Alternatively, the visible feature 1406, 1406a may be realised through the application of an additive, such as an indelible colourant; or one or more layers, each having a visually distinguishing feature that is secured onto the exterior surface of the elongate member or an exterior surface of the surgical tool 1402, 1402a.

During manufacture, the visible feature 1406, 1406a and the effector 1404, 1404a are arranged to be in a pre-defined alignment, such that the location of the visible feature 1406, 1406a is fixed relative to a roll orientation of the effector 1404, 1404a. For instance, the effector 1404 is a gripper with two arms, with each arm spaced 180° apart. The visible feature 1406 is a longitudinal line that is located along the exterior surface of the surgical tool 1402 at 90° from either of the two arms. Should this longitudinal line be seen during an operation, it will provide an indication of the orientation of the effector 1404. The effector 1404, 1404a rotates together with the visible feature 1406, 1406a.

Should the visible feature 1406, 1406a be an indicator of the roll orientation of the effector 1404, 1404a, the visible feature 1406, 1406a may be disposed either adjacent to the effector 1404, 1404a; adjacent to where the elongate member couples to the surgical tool 1402, 1402a; or both. This is because while the effector 1404, 1404a is translatable, the location of a camera that is used to monitor the effector 1404, 1404a is fixed. Should the effector 1404, 1404a translate away from the camera, the effector 1404, 1404a may no longer be seen clearly from images fed by the camera. In this scenario, the elongate member will then be in the camera view, whereby the position of the visible feature 1406, 1406a that is provided adjacent to where the elongate member couples to the surgical tool 1402, 1402a will then provide an indication of the orientation of the effector 1404, 1404a. Similarly, an adjacent placement of the visible feature 1406, 1406a to the effector 1404, 1404a provides for an indication of the orientation of the effector 1404, 1404a in the scenario where it is important where the tip of the effector 1404, 1404a is facing. When the effector 1404, 1404a is an electrocautery probe, its tip may not be clearly seen from the camera even if the electrocautery probe is in the camera view. Thus, the position of the visible feature 1406, 1406a that is provided adjacent to the effector 1404, 1404a will then provide an indication of the orientation of the effector 1404, 1404a.

The above mentioned camera provides a user viewing location with a defined user field of view disposed sufficiently adjacent to the distal end of the surgical tool 1402, 1402a such that the user is able to observe the visible feature 1406, 1406a of the elongate member and/or the surgical tool 1402, 1402a. In an embodiment, the camera may be attached to an auxiliary guide lumen and has a fixed position and orientation that is offset from the distal end of the guide lumen. The user field of view may be a display device such as a computer screen positioned in close proximity to the user.

When the surgical tool 1402, 1402a and the effector 1404, 1404a are passed through from the proximate end to the distal end of the elongate member, the surgical tool 1402, 1402a and the effector 1404, 1404a may be rolled ten degrees off with respect to the elongate member. In other words, it is difficult to ensure that the surgical tool and effector are not rolled or twisted during manual insertion due to the flexibility of the surgical tool 1402, 1402a and the effector 1404, 1404a. When the surgical tool 1402, 1402a and the effector 1404, 1404a emerge from the distal end of the elongate member, the position of the visible feature 1406, 1406a provided on the elongate member, the surgical tool 1402, 1402a or both serves to provide the degree of roll of the surgical tool 1402, 1402a and the effector 1404, 1404a with respect to the elongate member.

The visible feature 1406, 1406a may also serve to indicate to the user that the effector 1404, 1404a is in a correct orientation at the medical site in order to carry out the endoscopy procedure effectively. For example, the effector 1404 may consist of three gripper arms and the visible feature 1406 may be a red colour mark adjacent to one of the three gripper arms. The user requires that that specific gripper arm be aligned at ninety degrees in the user field of view before carrying out the endoscopy procedure. After the gripper arms are extended through the elongate member, the user is able to see the gripper arms but the visible feature 1406 (i.e. the red mark) is not visible in the user's field of view (i.e. the gripper arms are rotated past the ideal position). The user then rotates the effector 1404 such that the red mark is aligned at ninety degrees in the user's field of view. In addition, the visible feature 1406 may also serve to indicate that the gripper arms rotate in a correct direction in accordance with the user's input at an effector instrument panel.

In an embodiment, the visible feature 1406, 1406a may be provided on either the elongate member or the surgical tool 1402 or both, such that the visible feature 1406, 1406a is located at a predefined length from the effector 1404, 1404a, so that the visible feature 1406, 1406a provides a measure of translation of the effector 1404, 1404a. Translation orientation of the effector 1404, 1404a may be required due to manufacturing tolerances of the effector 1404, 1404a and the elongate member.

Further, the visible feature 1406, 1406a may also serve to indicate to the user that the effector 1404, 1404a is at a correct translation length at the medical site in order to carry out the endoscopy procedure effectively. For example, the effector may consist of an electrocautery probe and is required to be extended by two metres away from the elongate member before carrying out the endoscopy procedure. In this case, the visible feature may be a red colour mark located at the two metre mark of the surgical tool. After the electrocautery probe is extended through the elongate member, the user is able to see the elongate member and the electrocautery probe but the visible feature (i.e. the red mark) is not in the user's field of view (i.e. the electrocautery probe is not at the ideal position). The user then adjusts the electrocautery probe such that the red mark is visible in the user's field of view. In addition, the visible feature may also serve to indicate that the electrocautery probe translates in a correct direction in accordance with the user's input at an effector instrument panel.

The visible feature 1406, 1406a indicating the roll orientation of the effector 1404, 1404a and the visible feature indicating the measure of translation of the effector 1404, 1404a may be separate visible features that are visually distinguishable from each other. The visible feature 1406, 1406a indicating the translation and the roll of the effector 1404, 1404a may be formed by any one or more of an indelible colourant, laser marking, embossing or surface texturing. The visible features 1406, 1406a formed by indelible colorant may be of a different colour from the elongate member and/or the surgical tool 1402, 1402a. Further, the visible feature 1406, 1406a may be any one or more of a shape, a symbol or text and may be part of a pattern provided on the elongate member and/or the surgical tool. The pattern is also visibly distinguishable from the remainder of the exterior surface of the elongate member and the exterior surface of the surgical tool 1402, 1402a. Such a pattern includes one or more sets of such visible features 1406, 1406a, whereby one set is used as an indicator of the roll orientation of the effector 1404, 1404a, while another set is used as an indicator of the measure of translation of the effector 1404, 1014a.

The visible feature 1406, 1406a may also be made to maximize visibility of the feature against the appearance of the surroundings such as color, brightness, texture, specularity, or reflectivity. Preferably, the visible feature 1406, 1406a may be durable against chemical attack or mechanical abrasion and consist of implant grade biocompatible materials in the event that performing the medical procedure contains a risk of dislodging material from the visible feature.

For example, the visible feature 1406, 1406a indicating the roll orientation of the effector 1404, 1404a may be the letter "A" that is laser marked and embossed in blue while the visible feature indicating the measure of translation of the effector 1404, 1404a may be the symbol "$\Omega$" that is protruded in red using an indelible colourant. In an embodiment whereby the visible feature is a pattern, the pattern may be a series of continuous protrusions along the elongate member and extending into the surgical tool 1402, 1402a and effector 1404, 1404a. Further, the visible features 1406, 1406a indicating the translation and the roll of the effector 1404, 1404a may be partially obstructed by the effector 1404, 1404a but may still provide an indication of the roll or translation orientation of the effector 1404, 1404a. For example, the visible feature may be the letter "A" laser marked on the effector 1404, 1404a. Even if the lower part of the letter "A" is covered, the upper part of the letter may still indicate the direction of orientation of the effector 1404, 1404a.

The visible features 1406, 1406a indicating the translation and the roll of the effector 1404, 1404a may include a secondary feature that is visible in the user's field of view to indicate a correct translation and rotation of the effector 1404, 1404a. The secondary feature may be part of the primary visible feature or may be a separate feature. The presence of the secondary feature may be advantageous as the user does not rely on only one critical portion of the feature or only a single visible feature to indicate correct alignment.

In an example, the primary visible feature to indicate that the effector is in the correct roll orientation is the letter "A". In the event that the effector is already aligned at a desired position but the letter "A" is completely obscured by surrounding tissue or other surgical instrument, the secondary feature (e.g. an embossed star symbol) located adjacent to the letter "A" may serve to indicate that the effector is correctly aligned.

In an embodiment, an endoscopy system may comprise the endoscopy apparatus 1400 as described above and may further include a drive mechanism coupled to operate the endoscopy apparatus 1400. The endoscopy system may also include an endoscopy surgical instrument controller to control the drive mechanism and the endoscopy surgical instrument controller may be configured to send a signal prompting for alignment of the roll orientation of the effector 1404, 1404a to be performed; receive a response that the alignment is completed; and grant operation access to the effector 1404, 1404a of the endoscopy apparatus 1400.

Access to the effector 1404, 1404a may only be allowed after the alignment of the surgical tool 1402, 1402a is determined to be satisfactory. This acts as a safety mechanism so that the effector 1404, 1404a may not be activated and used which may harm the patient when it is at an unsatisfactory position.

It will be appreciated by a person skilled in the art that numerous variations and/or modifications may be made to the present invention as shown in the embodiments without departing from a spirit or scope of the invention as broadly described. The embodiments are, therefore, to be considered in all respects to be illustrative and not restrictive.

**ITEMS**

1. An endoscopy apparatus comprising:

an elongate member for insertion into a shaft of a transport endoscope,
a surgical tool coupled to a distal end of the elongate member, the surgical tool having an effector at an opposite end; and
a visible feature provided on the elongate member, the surgical tool or both, the location of the visible feature being fixed relative to a roll orientation of the effector, so that a position of the visible feature during use indicates the roll orientation of the effector.

2. The endoscopy apparatus of item 1, further comprising a visible feature provided on either the elongate member, the surgical tool or both, wherein the visible feature is located a predefined length from the effector, so that during use the visible feature provides a measure of translation of the effector.

3. The endoscopy apparatus of item 2, wherein the visible feature indicating the roll orientation of the effector and the visible feature providing a measure of translation of the effector are separate visible features.

4. The endoscopy apparatus of any one of the preceding items, wherein the visible feature provided on the elongate member and indicating the roll orientation of the effector is located adjacent to where the elongate member couples to the surgical tool and wherein the visible feature provided on the surgical tool and indicating the roll orientation of the effector is located adjacent to the effector.

5. The endoscopy apparatus of any one of the preceding items, wherein the visible feature extends over a portion along a length of the elongate member, the surgical tool or both.

6. The endoscopy apparatus of any one of the preceding items, wherein the visible feature extends over a portion along a cross-sectional perimeter of the elongate member, the surgical tool or both.

7. The endoscopy apparatus of any one of the preceding items, wherein the visible feature is formed by any one or more of: indelible colourant; laser marking, embossing or surface texturing.

8. The endoscopy apparatus of item 7, wherein the indelible colorant is of a different colour from the elongate member, the surgical tool or both.

9. The endoscopy apparatus of any one of the preceding items, wherein the visible feature comprises any one or more of a shape, a symbol or text.

10. The endoscopy apparatus of any one of the preceding items, wherein the visible feature is part of a pattern provided on the elongate member, the surgical tool or both.

11. The endoscopy surgical instrument of any one of the preceding items, wherein the effector comprises any one of an arm, a gripper or an electrocautery probe.

12. An endoscopy system comprising

the endoscopy apparatus of any of the items 1 to 11;
a drive mechanism coupled to operate the endoscopy apparatus; and
an endoscopy surgical instrument controller to control the drive mechanism, the endoscopy surgical instrument controller configured to

send a signal prompting for alignment of the roll orientation of the effector to be performed;
receive a response that the alignment is completed; and

grant operation access to the effector of the endoscopy apparatus.

13. An endoscopy surgical instrument controller for an endoscopy surgical instrument, the endoscopy surgical instrument comprising

a driving motor;
a following motor;
a joint arrangement;
a pulling tendon that couples the driving motor to the joint arrangement; and
a pushing tendon that couples the following motor to the joint arrangement, wherein the joint arrangement is actuated by the driving motor withdrawing the pulling tendon and the following motor releasing the pushing tendon,

the endoscopy surgical instrument controller comprising:

at least one processor; and
at least one memory including computer program code, the at least one memory and the computer program code configured to, with the at least one processor, cause the endoscopy surgical instrument controller at least to:

establish a displacement range occurring at the pulling tendon, within which the pulling tendon experiences maximum tension from being withdrawn by the driving motor;
determine whether a command received to actuate the joint arrangement causes the driving motor to withdraw a length of the pulling tendon that falls within the displacement range; and
instruct the following motor to restrict the releasing of the pushing tendon when the command is received, whereby a length of the pushing tendon released by the following motor is less than a length of the pulling tendon withdrawn by the driving motor over the displacement range, so that the tension experienced in the pulling tendon is caused by an extension of the pulling tendon.

14. The endoscopy surgical instrument controller of item 13, wherein the endoscopy surgical instrument controller is further configured to
instruct either the driving motor to have the length of the pulling tendon withdrawn or the following motor to have the length of the pushing tendon released be in accordance with a scaling factor to each other during at least a portion of the operation of the driving motor and the following motor.

15. The endoscopy surgical instrument controller of item 13 or 14, wherein the scaling occurs over the entire displacement range.

16. The endoscopy surgical instrument controller of item 14, wherein the scaling factor is at unity for at least a portion of the operation of the driving motor and the following motor.

17. The endoscopy surgical instrument controller of any one of the items 13, 14 and 16, wherein the endoscopy surgical instrument controller is further configured to
instruct the following motor to prevent release of the pushing tendon after the command is detected.

18. The endoscopy surgical instrument controller of any one of the items 13, 14, 16 and 17, wherein the endoscopy surgical instrument controller is further configured to
operate the driving motor and the following motor so that the length of the pulling tendon withdrawn is approximately the same as the length of the pushing tendon released before the command is detected.

19. The endoscopy surgical instrument controller of any one of the items 13 to 18, wherein the pulling tendon and the pushing tendon of the endoscopy surgical instrument Is a singular piece.

20. The endoscopy surgical instrument controller of any one of the items 13 to 19, wherein the endoscopy surgical instrument controller is further configured to:
detect for stoppage of the driving motor to establish a point within the displacement range where maximum tension is experienced by the pulling tendon.

21. An endoscopy system comprising:

an endoscopy surgical instrument comprising:

a driving motor;
a following motor;
a joint arrangement;
a pulling tendon that couples the driving motor to the joint arrangement; and
a pushing tendon that couples the following motor to the joint arrangement, wherein the joint arrangement is actuated by the driving motor withdrawing the pulling tendon and the following motor releasing the pushing tendon; and

an endoscopy surgical instrument controller coupled to the endoscopy surgical instrument, the endoscopy surgical instrument controller configured to:

establish a displacement range occurring at the pulling tendon, within which the pulling tendon experiences maximum tension from being withdrawn by the driving motor;

determine whether a command received to actuate the joint arrangement causes the driving motor to withdraw a length of the pulling tendon that falls within the displacement range; and

instruct the following motor to restrict the releasing of the pushing tendon when the command is received, whereby a length of the pushing tendon released by the following motor is less than a length of the pulling tendon withdrawn by the driving motor over the displacement range, so that the tension experienced in the pulling tendon is caused by an extension of the pulling tendon.

22. The endoscopy system of item 21, wherein the endoscopy surgical instrument controller is further configured to instruct either the driving motor to have the length of the pulling tendon withdrawn or the following motor to have the length of the pushing tendon released be in accordance with a scaling factor to each other during at least a portion of the operation of the driving motor and the following motor.

23. The endoscopy system of item 21 or 22, wherein the scaling occurs over the entire displacement range.

24. The endoscopy system of item 22, wherein the scaling factor is at unity for at least a portion of the operation of the driving motor and the following motor.

25. The endoscopy system of any one of the items 21, 22 and 24, wherein the endoscopy surgical instrument controller is further configured to

instruct the following motor to prevent release of the pushing tendon after the command is detected.

26. The endoscopy system of any one of the items 21, 22, 24 and 25, wherein the endoscopy system is further configured to

operate the driving motor and the following motor so that the length of the pulling tendon withdrawn is approximately the same as the length of the pushing tendon released before the command is detected.

27. The endoscopy system of any one of the items 21 to 26, wherein the pulling tendon and the pushing tendon of the endoscopy surgical instrument Is a singular piece.

28. The endoscopy system of any one of the items 21 to 27, wherein the endoscopy surgical instrument controller is further configured to:

detect for stoppage of the driving motor to establish a point within the displacement range where maximum tension is experienced by the pulling tendon.

29. An endoscopy surgical instrument controller of an endoscopy system, the endoscopy system comprising an endoscopy surgical instrument, the endoscopy surgical instrument comprising

a drive mechanism; and

a terminal joint actuated by the drive mechanism, the terminal joint being disposed at the distal end of the endoscopy surgical instrument;

the endoscopy system further comprising

an input device in electrical communication with the drive mechanism, whereby movement of the input device causes the actuation of the terminal joint,

the endoscopy surgical instrument controller comprising:

at least one processor; and

at least one memory including computer program code, the at least one memory and the computer program code configured to, with the at least one processor, cause the endoscopy surgical instrument controller at least to:

detect for a signal resulting from movement of the input device, the signal providing a Cartesian position in a master workspace to which the input device has been moved, the master workspace providing a boundary within which the input device can be moved;

process the received Cartesian position against a database that comprises Cartesian positions for the master workspace; Cartesian positions for a slave workspace providing a boundary within which the terminal joint can be actuated; and a mapping table that maps each Cartesian position in the master workspace to at least one Cartesian position in the slave workspace;
determine a matching Cartesian position in the slave workspace for the received Cartesian position; and command the drive mechanism to actuate the terminal joint to the matching Cartesian position in the slave workspace.

30. The endoscopy surgical instrument controller of item 29, wherein a plurality of the Cartesian positions in the master workspace is mapped to a Cartesian position in the slave workspace.

31. The endoscopy surgical instrument controller of item 30, wherein the Cartesian position in the slave workspace to which the plurality of the Cartesian positions in the master workspace is mapped provides a closest matching Cartesian position in the slave workspace for each of the plurality of the Cartesian positions in the master workspace.

32. An endoscopy surgical instrument controller of an endoscopy system, the endoscopy system comprising an endoscopy surgical instrument, the endoscopy surgical instrument comprising

a drive mechanism; and
a terminal joint actuated by the drive mechanism, the terminal joint being disposed at the distal end of the endoscopy surgical instrument;

the endoscopy system further comprising
an input device in electrical communication with the drive mechanism, whereby movement of the input device causes the actuation of the terminal joint,
the endoscopy surgical instrument controller comprising:

at least one processor; and
at least one memory including computer program code, the at least one memory and the computer program code configured to, with the at least one processor, cause the endoscopy surgical instrument controller at least to:

create a mobile tracer inside a slave workspace providing a boundary within which the terminal joint can be actuated, the mobile tracer being configured to track the input device by shifting inside the slave workspace in response to the input device being moved;
detect for a signal resulting from movement of the input device;
shift the mobile tracer to a Cartesian position within the slave workspace, wherein a distance of the shift depends on a Cartesian position of the input device inside a master workspace before and after the movement of the input device, the master workspace providing a boundary within which the input device can be moved; and
command the drive mechanism to actuate the terminal joint to the Cartesian position of the mobile tracer in the slave workspace after the shift.

33. The endoscopy surgical instrument controller of item 32, wherein the endoscopy surgical instrument controller is further configured to interrogate a database in which the slave workspace and the master workspace are stored when creating the mobile tracer.

34. The endoscopy surgical instrument controller of item 32 or 33, wherein the endoscopy surgical instrument controller is further configured to
link the input device to the mobile tracer when configuring the mobile tracer to track the input device.

35. The endoscopy surgical instrument controller of item 34, wherein the mobile tracer moves adjacent to or along a perimeter of the slave workspace when the input device moves within a region of the master workspace that is outside of the slave workspace.

36. The endoscopy surgical instrument controller of any one of the items 29 to 35, wherein

the endoscopy system further comprises
a feedback force module coupled to the input device, the feedback force module configured to produce a resistive force that keeps the input device within a region of the master workspace that corresponds to inside the boundary of the slave workspace,
wherein the endoscopy surgical instrument controller is further configured to
transmit a signal to the feedback force module to increase the resistive force the further the input device moves outside of the region of the master workspace that corresponds to the boundary of the slave workspace.

37. The endoscopy surgical instrument controller of item 36, wherein the endoscopy surgical instrument controller is further configured to

compute a magnitude of the increase of the resistive force produced by the feedback force module; and
transmit a command to the driving motor or the drive mechanism to adjust a torque limit applied to actuate the joint arrangement or the terminal joint in response to the computed magnitude of the increase of the resistive force.

38. The endoscopy surgical instrument controller of any one of the items 29 to 37, wherein the Cartesian position provides a location in three-dimensional space.

39. The endoscopy surgical instrument controller of any one of the items 29 to 38, wherein the master workspace has a larger volume than the slave workspace.

40. The endoscopy surgical instrument controller of any one of the items 29 to 39, wherein the endoscopy surgical instrument further comprises an effector coupled to the terminal joint, wherein the effector is any one of an arm, a gripper or an electrocautery probe.

41. The endoscopy surgical instrument controller of any one of the items 29 to 40, wherein the endoscopy surgical instrument controller is further configured to:
synchronise an orientation of the input device with an orientation of the joint arrangement or the terminal joint, such that a change of orientation of the input device results in a corresponding change in orientation of the joint arrangement or the terminal joint.

42. An adaptor for coupling a motor shaft to actuate a tendon of an endoscopy surgical instrument, the adaptor comprising

a housing;
a drum around which the tendon winds, the drum being rotatably coupled to the housing; and
an energy storage mechanism arranged to apply torque on the drum.

43. The adaptor of item 42, wherein the energy storage mechanism is positioned so as to apply the torque in a direction that winds the tendon around the drum.

44. The adaptor of item 42 or 43, wherein one end of the energy storage mechanism is coupled to the drum and an opposite end of the energy storage mechanism is coupled to the housing.

45. The adaptor of any one of the preceding items, wherein the energy storage mechanism is disposed around a portion of the drum.

46. The adaptor of any one of the items 42 to 44, wherein the energy storage mechanism is disposed at either end of the drum.

47. The adaptor of any one of the items 42 to 46, wherein the energy storage mechanism is designed so that the applied torque causes a tension of around 0.5 to 3N in the tendon.

48. The adaptor of any one of the items 42 to 47, wherein the energy storage mechanism comprises a resiliently flexible member or a hydraulic device.

49. The adaptor of item 48, wherein the resiliently flexible member is a torsion spring.

50. The adaptor of item 49, wherein the torsion spring has any one of the following configurations: flat spiral, coil or axially twisted.

51. The adaptor of item 50, wherein the torsion spring of the coil configuration has a fineness ratio of at least 14.

52. The adaptor of any one of the items 42 to 51, further comprising at least one groove to which the tendon engages, the at least one groove being provided on a portion of the drum.

53. The adaptor of item 52, wherein the groove extends along the diameter of the drum.

54. The adaptor of item 52 or 53, further comprising a screw thread formed on the drum, the screw thread providing a plurality of the grooves to which the tendon engages.

55. A transport endoscope docking station comprising:

a base having an endoscope attachment surface for mounting a transport endoscope, the base further having a drive mechanism attachment surface for mounting a drive mechanism to actuate a robotic member carried by the transport endoscope; and
a platform to which the base is rotatably coupled.

56. The transport endoscope docking station of item 55, further comprising a rotary mechanism arranged to facilitate rotation of the base relative to the platform.

57. The transport endoscope docking station of item 56, wherein the rotary mechanism is disposed between the base and the platform.

58. The transport endoscope docking station of item 57, wherein the rotary mechanism is disposed between the base and a portion of the platform that is adjacent to the drive mechanism attachment surface of the base, between the base and a portion of the platform that is adjacent to the endoscope attachment surface of the base, or both.

59. The transport endoscope docking station of any one of the items 56 to 58, wherein the rotary mechanism is any one or more of a ball bearing arrangement, a roller bearing arrangement and a lubricated washer arrangement.

60. The transport endoscope docking station of any one of the items 55 to 59, further comprising a locking mechanism arranged to lock the rotation of the base relative to the platform.

61. The transport endoscope docking station of item 60, further comprising a connector that couples the locking mechanism to the base.

62. The transport endoscope docking station of item 61, wherein the connector comprises any one of: a timing belt arrangement, a gear arrangement or an arm linkage.

63. The transport endoscope docking station of item 59, wherein the locking mechanism comprises an electrically activated device configured to lock the base through frictional engagement.

64. The transport endoscope docking station of item 63, wherein the locking mechanism is configured to lock the base by default.

65. The transport endoscope docking station of any one of the items 60 to 64, wherein the locking mechanism comprises any one or more of a brake pad, a clamp and a latch and vault arrangement.

66. The transport endoscope docking station of any one of the items 61 to 65, further comprising a damping mechanism to dampen rotation of the base.

67. The transport endoscope docking station of item 66, wherein the damping mechanism comprises a rotary damper having any one of the following configurations: a rotary friction disk, a rotary friction gear rack, a pneumatic rotary damper or visco-elastic.

68. The transport endoscope docking station of item 67, wherein the rotary damper is coupled to the connector coupling the locking mechanism to the base.

69. The transport endoscope docking station of any one of the items 55 to 68, wherein an axis running through a centre of an end of the adaptor removably attached to the base is aligned with the rotation axis of the base.

70. The transport endoscope docking station of any one of the items 55 to 69, wherein the base further comprises a stand to which an actuator assembly of the drive mechanism is coupled, the actuator assembly comprising at least one actuator to actuate the robotic member.

71. The transport endoscope docking station of item 70, wherein the actuator assembly comprises an adaptor attachment surface to which an adaptor is coupled, the adaptor for coupling the actuator to the robotic member.

72. The transport endoscope docking station of any one of the items 55 to 71, wherein at least a portion of the drive mechanism is integral with the base and a remainder of the drive mechanism is removably attachable to the integrated portion of the drive mechanism.

## Claims

1. An endoscopy surgical instrument controller of an endoscopy system, the endoscopy system comprising an endoscopy surgical instrument, the endoscopy surgical instrument comprising

   - a drive mechanism; and
   - a terminal joint actuated by the drive mechanism, the terminal joint being disposed at the distal end of the endoscopy surgical instrument;
   the endoscopy system further comprising
   an input device in electrical communication with the drive mechanism, whereby movement of the input device causes the actuation of the terminal joint,
   the endoscopy surgical instrument controller comprising:

   - at least one processor; and
   - at least one memory including computer program code, the at least one memory and the computer program code configured to, with the at least one processor, cause the endoscopy surgical instrument controller at least to:

     - detect for a signal resulting from movement of the input device, the signal providing a Cartesian position in a master workspace to which the input device has been moved, the master workspace providing a boundary within which the input device can be moved;
     - process the received Cartesian position against a database that comprises Cartesian positions for the master workspace; Cartesian positions for a slave workspace providing a boundary within which the terminal joint can be actuated; and a mapping table that maps each Cartesian position in the master workspace to at least one Cartesian position in the slave workspace;
     - determine a matching Cartesian position in the slave workspace for the received Cartesian position; and
     - command the drive mechanism to actuate the terminal joint to the matching Cartesian position in the slave workspace.

2. The endoscopy surgical instrument controller of claim 1, wherein a plurality of the Cartesian positions in the master workspace is mapped to a Cartesian position in the slave workspace.

3. The endoscopy surgical instrument controller of claim 2, wherein the Cartesian position in the slave workspace to which the plurality of the Cartesian positions in the master workspace is mapped provides a closest matching Cartesian position in the slave workspace for each of the plurality of the Cartesian positions in the master workspace.

4. An endoscopy surgical instrument controller of an endoscopy system, the endoscopy system comprising an endoscopy surgical instrument, the endoscopy surgical instrument comprising

   - a drive mechanism; and
   - a terminal joint actuated by the drive mechanism, the terminal joint being disposed at the distal end of the endoscopy surgical instrument;

the endoscopy system further comprising
an input device in electrical communication with the drive mechanism, whereby movement of the input device causes the actuation of the terminal joint,
the endoscopy surgical instrument controller comprising:

- at least one processor; and
- at least one memory including computer program code, the at least one memory and the computer program code configured to, with the at least one processor, cause the endoscopy surgical instrument controller at least to:

- create a mobile tracer inside a slave workspace providing a boundary within which the terminal joint can be actuated, the mobile tracer being configured to track the input device by shifting inside the slave workspace in response to the input device being moved;
- detect for a signal resulting from movement of the input device;
- shift the mobile tracer to a Cartesian position within the slave workspace, wherein a distance of the shift depends on a Cartesian position of the input device inside a master workspace before and after the movement of the input device, the master workspace providing a boundary within which the input device can be moved; and
- command the drive mechanism to actuate the terminal joint to the Cartesian position of the mobile tracer in the slave workspace after the shift.

5. The endoscopy surgical instrument controller of claim 4, wherein the endoscopy surgical instrument controller is further configured to interrogate a database in which the slave workspace and the master workspace are stored when creating the mobile tracer.

6. The endoscopy surgical instrument controller of claim 4 or 5, wherein the endoscopy surgical instrument controller is further configured to
link the input device to the mobile tracer when configuring the mobile tracer to track the input device.

7. The endoscopy surgical instrument controller of claim 6, wherein the mobile tracer moves adjacent to or along a perimeter of the slave workspace when the input device moves within a region of the master workspace that is outside of the slave workspace.

8. The endoscopy surgical instrument controller of any one of the claims 1 to 7,

wherein the endoscopy system further comprises
a feedback force module coupled to the input device, the feedback force module configured to produce a resistive force that keeps the input device within a region of the master workspace that corresponds to inside the boundary of the slave workspace,
wherein the endoscopy surgical instrument controller is further configured to
transmit a signal to the feedback force module to increase the resistive force the further the input device moves outside of the region of the master workspace that corresponds to the boundary of the slave workspace.

9. The endoscopy surgical instrument controller of claim 8, wherein the endoscopy surgical instrument controller is further configured to

- compute a magnitude of the increase of the resistive force produced by the feedback force module; and
- transmit a command to the driving motor or the drive mechanism to adjust a torque limit applied to actuate the joint arrangement or the terminal joint in response to the computed magnitude of the increase of the resistive force.

10. The endoscopy surgical instrument controller of any one of the claims 1 to 9, wherein the Cartesian position provides a location in three-dimensional space.

11. The endoscopy surgical instrument controller of any one of the claims 1 to 10, wherein the master workspace has a larger volume than the slave workspace.

12. The endoscopy surgical instrument controller of any one of the claims 1 to 11, wherein the endoscopy surgical instrument further comprises an effector coupled to the terminal joint, wherein the effector is any one of an arm, a gripper or an electrocautery probe.

13. The endoscopy surgical instrument controller of any one of the claims 1 to 12, wherein the endoscopy surgical instrument controller is further configured to:
synchronise an orientation of the input device with an orientation of the joint arrangement or the terminal joint, such that a change of orientation of the input device results in a corresponding change in orientation of the joint arrangement or the terminal joint.

**Figure 1**

Figure 2

300

**Distal End**

Joint 306

304

Tendon 302

Sheath 312

314

316

318

Driving Motor 308

Following Motor 310

Proximal End

**Figure 3A**

---

**Distal End**

Joint

306

302

Tendon 304

Sheath 312

314

316

318

Following Motor 310

Driving Motor 308

Proximal End

**Figure 3B**

EP 4 183 315 A1

Start

Compute reference
position command         402

Send reference
position to the driving
motor                    404

Send reference
position to the following
motor                    404

**Figure 4A**

(PRIOR ART)

Start

Compute reference
position command         408

Send reference
position to the driving
motor                    410

Send the current position
of the driving motor as
reference position to the   412
following motor

**Figure 4B**

Figure 5A

Figure 5B

EP 4 183 315 A1

**Figure 6**

EP 4 183 315 A1

Figure 7

Figure 8

Figure 9

**Figure 10**

EP 4 183 315 A1

End
Effector

1106

Tendon is
slack

1002

Tendon
remains taut

Drum
releases
tendon
when
instrument
is detached

1100

1100

1000A

1000B

Energy
storage
mechanism
provides
tension to
tendon when
instrument is
detached

Drum
actuator 1

Drum
actuator 2

**Figure 11A**

**Figure 11B**

Figure 12

Figure 13

**Figure 14**

Europäisches
Patentamt

European
Patent Office

Office européen
des brevets

# EUROPEAN SEARCH REPORT

Application Number

EP 23 15 0127

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/106493 A1 (NIEMEYER GUNTER D [US] ET AL) 18 May 2006 (2006-05-18) * paragraphs [0021], [0120] – [0122], [0152], [0153], [0170], [0171] * * figures 1A, 1B * ----- | 1-13 | INV. A61B1/00 A61B1/005 A61B1/018  ADD. A61B18/00 A61B18/14 A61B34/00 A61B90/00 |

TECHNICAL FIELDS
SEARCHED      (IPC)

A61B

The present search report has been drawn up for all claims

| Place of search | Date of completion of the search | Examiner |
|---|---|---|
| Munich | 30 March 2023 | Hemb, Björn |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 ..........................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 15 0127

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2023

| Patent document cited in search report | | Publication date | Patent family member(s) | | Publication date |
|---|---|---|---|---|---|
| US 2006106493 | A1 | 18-05-2006 | AT | 305639 T | 15-10-2005 |
| | | | DE | 60022911 T2 | 22-06-2006 |
| | | | EP | 1269389 A1 | 02-01-2003 |
| | | | ES | 2248066 T3 | 16-03-2006 |
| | | | US | 6424885 B1 | 23-07-2002 |
| | | | US | 2003004610 A1 | 02-01-2003 |
| | | | US | 2004039485 A1 | 26-02-2004 |
| | | | US | 2006106493 A1 | 18-05-2006 |
| | | | WO | 0060521 A1 | 12-10-2000 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015142290 A

- WO 2017048194 A